# EUROPEAN PATENT SPECIFICATION

(11) **EP 2 384 112 B1**
(45) Date of publication and mention of the grant of the patent: **27.02.2019**
(21) Application number: 09836460.7
(22) Date of filing: 30.12.2009
(51) Int. Cl.: A01N 1/02, A61K 31/135, A61K 31/46, A61K 31/55, A61K 31/137, A61K 31/198, A61K 31/573, A61K 38/11, A61P 43/00

(54) **A COMPOSITION COMPRISING NOR-ADRENALINE AND A NET INHIBITOR FOR ADMINISTERING TO A BRAIN-DEAD, HEART-BEATING POTENTIAL ORGAN DONOR.**
ZUSAMMENSETZUNG, DIE NORADRENALIN UND EINEN NET-HEMMER ENTHÄLT, ZUR VERABREICHUNG AN EINEN HIRNTOTEN HEART-BEATING POTENTIELLEN ORGANSPENDER
COMPOSITION COMPRENANT DE LA NORADRÉNALINE ET UN INHIBITEUR DU NET DESTINÉE À ÊTRE ADMINISTRÉE À UN DONNEUR D'ORGANE POTENTIEL EN ÉTAT DE MORT CÉRÉBRALE DONT LE COEUR BAT TOUJOURS

(30) Priority: 30.12.2008 SE 0802678; 19.03.2009 US 210444 P
(43) Date of publication of application: 09.11.2011
(73) Proprietor: XVIVO Perfusion AB, 412 51 Göteborg (SE)
(72) Inventor: STEEN, Stig, S-224 77 Lund (SE)
(74) Representative: Henderson, Helen Lee
(86) International application number: PCT/SE2009/000541
(87) International publication number: WO 2010/077200

(56) References cited:
- WO-A1-01/85171
- WO-A2-2005/056763
- JOHN ET AL: "Donor management and selection for heart transplantation", SEMINARS IN THORACIC AND CARDIOVASCULAR SURGERY, SAUNDERS, PHILADELPHIA, PA, US, vol. 16, no. 4, 29 December 2004 (2004-12-29), pages 364-369, XP005939963, ISSN: 1043-0679, DOI: 10.1053/J.SEMTCVS.2004.09.003
- ZAKY AHMED ET AL: "Hemodynamic and metabolic efficacy of dopamine versus norepinephrine in a brain-dead swine model.", LIVER TRANSPLANTATION : OFFICIAL PUBLICATION OF THE AMERICAN ASSOCIATION FOR THE STUDY OF LIVER DISEASES AND THE INTERNATIONAL LIVER TRANSPLANTATION SOCIETY SEP 2008 LNKD- PUBMED:18756452, vol. 14, no. 9, September 2008 (2008-09), pages 1266-1272, XP002676941, ISSN: 1527-6473
- MUIR JUDITH K ET AL: "Cocaine potentiates the blood pressure and cerebral blood flow response to norepinephrine in rats", EUROPEAN JOURNAL OF PHARMACOLOGY, vol. 249, no. 3, 1993, pages 287-292, XP002676942, ISSN: 0014-2999
- JOHNSSON G ET AL: "Interaction studies between three antidepressant drugs (chlorimipramine, imipramine and zimelidine) and noradrenaline, tyramine and vagal stimulation on the heart rate and blood pressure in dogs", ACTA PHARMACOLOGICA ET TOXICOLOGICA 1979 DK, vol. 45, no. 3, 1979, pages 192-197, XP002676943, ISSN: 0001-6683
- BOHM M. ET AL: 'Evidence for Reduction of Norepinephrine Uptake Sites in the Failing Human Heart' JOURNAL OF THE AMERICAN COLLEGE OF CARDIOLOGY vol. 25, no. 1, 1995, pages 146 - 153, XP003026464
- BENTLEY G. A.: 'The effect of local anaesthetic and anti-adrenaline drugs on the response of sympathetically innervated smooth muscle preparations to electrical stimulation at different frequencies' BRITISH JOURNAL OF PHARMACOLOGY AND CHEMOTHERAPY vol. 27, 1966, pages 64 - 80, XP003026465
- KUCHERUK A. S.: 'Effect of noradrenalin on duration of local anesthesia' BULLETIN OF EXPERIMENTAL BIOLOGY AND MEDICINE vol. 54, no. 1, 1963, pages 761 - 762, XP003026466
- SCHNUELLE P. ET AL: 'Donor catecholamine use reduces acute allograft rejection and improves graft survival after cadaveric renal transplantation' KIDNEY INTERNATIONAL vol. 56, 1999, pages 738 - 746, XP003026467
- SCHULAK J. A. ET AL: 'Donor pretreatment with lidocaine decreases incidence of early renal dysfunction in cadaver kidney transplantation' TRANSPLANTATION PROCEEDINGS vol. 22, no. 2, 1990, pages 353 - 354, XP003026468
- WIJNEN R. M. H. ET AL: 'Donor treatment after pronouncement of brain death: a neglected intensive care problem' TRANSPLANT INTERNATIONAL vol. 4, 1991, pages 186 - 190, XP003026469
- WOOD K. E. ET AL: 'Management of the potential organ donor' TRANSPLANTATION REVIEWS vol. 21, 2007, pages 204 - 218, XP022292959

## Description

### FIELD OF INVENTION

The present invention relates to a method of handling a potential organ donor immediately after brain death and until organs are harvested, and a composition and an infusion solution.

### BACKGROUND OF THE INVENTION

It is well known that there is a great shortage of donor organs, which are suitable for transplantation. Various documents have been published in this field. A summary of some of these is as follows. John et al.: Donor Management and selection for heart transplantation", Seminars in thoracic and cardiovascular surgery, Saunders, Philadelphia, PA, US, vol. 16, no. 4, 29 December 2004 (2004-12-29) pages 364-369 discusses the severe shortage of available donor organs.

Zaky Ahmed et al.: Hemodynamic and Metabolic Efficacy of Dopamine Versus Norepinephrine in a Brain-dead Swine Model.", Liver Transplantation: Official Publication of the American Association for the Study of Liver Diseases and the International Liver Transplantation Society Sep 2008 LNKD- Pubmed: 18756452, vol. 14, no. 9, September 2008 (2008-09), pages 1266-1272 discloses an infusion solution for intravascular administration for treatment of a brain-dead, heart-beating respirated potential organ donor, wherein the infusion solution comprises nor-adrenaline.

Muir Judith K et al.: "Cocaine potentiates the blood pressure and cerebral blood flow response to norepinephrine in rats", European Journal of Pharmacology, vol. 249, no. 3, 1993, pages 287-292 relates to a study to determine whether cocaine alters the blood pressure or cerebral blood flow response to exogenous norepinephrine.

Johnsson G et al.: "Interaction studies between three antidepressant drugs (chlorimipramine, imipramine and zimelidine) and noradrenaline, tyramine and vagal stimulation on the heart rate an blood pressure in dogs", Acta Pharmacologica et Toxicologica 1979, DK vol. 45, no. 3, 1979, pages 192-197 reports the results of a study between 3 antidepressant drugs on the heart rate and blood pressure of dogs.

Schnuelle, Peter et al.: "Donor catecholamine use reduces acute allograft rejection and improves graft survival after cadaveric renal transplantation" Kidney International, Vol. 56 (1999), pp. 738-746 reports data that suggests that the use of catecholamines in post-mortal organ donors during intensive care results in immunomodulating effects and improves graft survival in long-term follow-up.

WO 2005/056763 is a publication relating to methods and compositions for the preservation of cells using glucosaminoglycans and derivatives thereof.

Hemodynamic instability during and after brain death of a heart-beating donor is often associated with the deterioration of graft viability, leading to organ exclusion.

There is a need for a method of treating the potential organ donor after brain death and before harvesting of organs, which decreases the rejection rate of organs that are harvested from such donor.

### SUMMARY OF THE INVENTION

Accordingly, an object of the present invention is to mitigate, alleviate or eliminate one or more of the above-identified deficiencies and disadvantages singly or in any combination.

According to an aspect of the invention, there is provided the use of an infusion solution for intravascular administration for treatment of a brain-dead, heart-beating, respirated potential organ donor, comprising: adrenaline, nor-adrenaline and a NET inhibitor as defined in claim 1.

The NET inhibitor is cocaine or a stimulating analogue thereof. NET inhibitors described herein include at least one tricyclic antidepressant included in the group comprising: Amitriptyline (ELAVIL); Clomipramine (ANAFRANIL); Doxepin (ADAPIN, SINEQUAN); Imipramine (TROFANIL); Trimipramine (SURMONTIL); Amoxapine (ASENDIN); Desipramine (NORPRAMIN); Maprotinile (LUDIOMIL); Nortriptyline (PAMELOR); and Protriptyline (VIVACTIL). Further NET inhibitors described include at least one agent included in the group comprising: Venlafaxin (EFFEXOR); Atomexetine (WELLBUTRIN); Duloxetine (CYMBALTA); Mirtacapine (REMERON); Norclomipramine; Oxaprotiline; Lofepramine; Reboxetine; Maprotiline; Nomifensine; Doxepin; Mianserin; Viloxazine; Mirtazapine; and Nisoxetine.

According to a further embodiment, the infusion solution may further comprise at least one of: hydrocortisone, thyroxin, insulin, triiodotyronine, a vasopressor agent, such as desmopressin, and methylprednisolone.

The NET inhibitor may be present in an amount of 0.2 to 5 times of the amount of nor-adrenaline. In the case of cocaine, the ratio between cocaine and nor-adrenaline may be about 1:1.

The infusion solution comprises the components mentioned above and a pharmaceutically acceptable medium, such as pure water, Ringer's acetate solution or physiological sodium chloride solution. The components may be dissolved in the pharmaceutically acceptable medium in an amount of about 1 mg of nor-adrenaline, and about 1 mg of cocaine in about 50 ml of fluid.

In a further aspect, there is provided a method for treatment of a brain dead, heart-beating, respirated potential organ donor, comprising: infusion of the infusion solution mentioned above, in an amount sufficient for maintaining a mean arterial pressure of about 60 mmHg.

In a still further aspect, there is provided a kit for intravenous administration for treatment of a brain dead, heart-beating, respirated potential organ donor, comprising: an infusion bag comprising an infusion solution as mentioned above; an infusion pump for pumping the infusion solution to a needle inserted into the vascular system of the donor for controlling the amount of infusion solution entered into the donor and tubings for interconnecting the bag, pump and needle. The kit may comprise several sets of infusion bags, infusion pumps and tubings. The kit may further comprise a computer for controlling the pump or pumps, whereby the computer is operated according to a predetermined control strategy.

In a yet further aspect, there is provided a method of infusing a solution into the circulation system of a brain dead, heart-beating, respirated potential organ donor, comprising: determining that the donor is brain dead; continuing or initating respiration for oxygenation of the blood; infusion of an infusion solution as mentioned above; and controlling the infusion by means of a computer.

### BRIEF DESCRIPTION OF THE DRAWINGS

Further objects, features and advantages of the invention will become apparent from the following detailed description of embodiments of the invention with reference to the drawings, in which:
Fig. 1 is a schematic view of a nerve terminal;
Figs. 2, 3 and 4 are diagrams showing blood pressures during treatment with the compositions according to embodiments;
Fig. 5 is a schematic diagram of a kit for administration of an infusion solution according to embodiments;
Fig. 6 is a schematic diagram of another kit for administration of infusion solutions according to embodiments.

### DETAILED DESCRIPTION OF EMBODIMENTS

Below, several embodiments of the invention will be described. These embodiments are described in illustrating purpose in order to enable a skilled person to carry out the invention and to disclose the best mode. However, such embodiments do not limit the scope of the invention. Moreover, certain combinations of features are shown and discussed. However, other combinations of the different features are possible within the scope of the invention.

Definitions: In the context of the present description and embodiments the following definitions apply: The term "cocaine analogue" is intended to mean an analogue, which acts in the same or a similar way as cocaine in preserving the organs in a brain-dead, heart-beating, respirated donor before harvesting of the organs. The term "Pharmaceutically acceptable" means a non-toxic material that does not decrease the effectiveness of the biological activity of the active ingredients. Such pharmaceutically acceptable buffers, carriers or excipients are well-known in the art, see for example Remington's Pharmaceutical Sciences, 18th edition, A.R Gennaro, Ed., Mack Publishing Company (1990) and handbook of Pharmaceutical Excipients, 3rd edition, A. Kibbe, Ed., Pharmaceutical Press (2000). The term "physiologically acceptable solution" means a solution that does not interfere substantially with the fluids in the body.

An object of the below described embodiments is to improve the outcome of organ harvesting and transplantation from a donor, which has been declared brain-dead but still has a beating heart and which is ventillated.

The process of becoming brain dead is a traumatic experience for the body and its organs. Thus, a thorough understanding of the endocrinal, hormonal and metabolic events before, during and after declaration of brain death would be of interest for determining intervention.

Brain death may be initiated by a massive necrosis of brain cells, which may be due to different causes. Such necrosis may result in increased osmotic pressure in the brain, resulting in water absorption via the blood-brain barrier. Since the scull cannot expand, the intracranial pressure rises considerably.

When the intracranial pressure exceeds the systolic blood pressure, the brain is exposed to an ischemic condition, because blood cannot enter the brain. The brain may react by increasing the heart rate and blood flow and by increasing the systemic vascular resistance. In addition, the adrenal gland increases the level of circulating adrenalin (epinephrine) and nor-adrenaline (nor-epinephrine). This is called the Cushing reflex.

The heart rate may increase by several hundred percent, to a maximum heart rate. The blood pressure may increase to above 200 mmHg. This massive reaction is also called the "catecholamine storm" or "sympathetic autonomous storm". The adrenaline and/or nor-adrenaline levels may increase by 70 times, as described in more detail below.

If this increase of systolic blood pressure is insufficient for delivering blood to the brain, the brain will maintain its ischemic state. However, the brain cannot sustain more than about 10 minutes without blood supply.

If the intracranial pressure, for example due to the increased osmotic pressure, rises to more than about 300 mmHg, the brain cannot withstand such high pressures but disintegrates. The end result will be a progressive brain swelling, and herniation of the hipocampal gyri with lateral pressure of the brainstem, with eventual loss of brainstem function and loss of spontaneous respiration. This may results in herniation of the brain stem through the foramen magnum.

Other causes of (brain) death may result in somewhat different processes.

In Sweden, brain death is defined as irreversible loss of function of the entire brain including the brainstem. There are several indicia of brain death, which are of less interest for the present embodiments. However, after brain death, there is no cerebral blood circulation and no spontaneous respiration. The body temperature should be above 33°C and there should be no drug intoxication.

After brain death, the brain, including the brain stem, cannot retain its function, because it is permanently damaged.

During the "catecholamine storm", the levels of adrenaline and nor-adrenaline may rise considerably. During experiments on dogs, an increase in blood concentration of adrenaline from about 0.40 to 75 nmol/l and an increase of nor-adrenaline from about 0.40 to 12 nmol/l were obtained within one to three minutes.

After occurrence of brain death, the hypothalamic-pituitary-adrenal axis is disrupted. However, necrosis is followed by a release of cytokines, especially IL-6, which stimulates the adrenal gland to produce adrenaline and nor-adrenaline. Eventually, the production of these inotropics will be reduced and after some 60 minutes, the levels of adrenaline and nor-adrenaline will be lower than normal. This will result in vasoplegia by loss of the sympathetic vasotonus.

The pituitary gland also produces antidiuretic hormone (ADH), or vasopressin, which acts on the kidneys in order to control the water resorption. ADH has a short half-life-time of about 15 minutes and a shortage of ADH will occur after some 60 minutes (with large individual variations). Depletion of ADH may result in diabetes insipidus, resulting in production of large quantities of urine in the order of several liters per hour. Unless replacement of fluid takes place, diabetes insipidus will result in hypovolemia, a further reduction of blood pressure and eventual loss of circulation, resulting in ischemic damage of all organs.

Moreover, the pituitary gland produces adrenocorticotropic hormone (ACTH), which stimulates secretion of glucocorticoids, which stimulates the synthesis of adrenaline and nor-adrenaline.

In addition, the pituitary gland produces thyroid-stimulating hormone (TSH), which stimulates the thyroid gland to secrete the hormones thyroxin (T4) and triiodotyronine (T3). Depletion of T4 and T3 may result in a change from aerobic to anaerobic metabolism in for example the heart, which may result in increased lactate and pyruvate levels.

Because the pituitary gland is dependent on the hypothalamus, the operation of the pituitary gland is reduced or ceases, resulting in decreased circulating levels of for example T3, T4, ADH, ACTH, cortisol and insulin. This results in impaired aerobic metabolism, increased anaerobic metabolism, depletion of high-energy phosphates and increased lactate production.

Side effects of high levels of catecholamines are tachycardia, atrial and ventricular arrhythmias as well as conduction abnormalities.

Pulmonary edema may result after high levels of catecholamines, especially adrenaline.

Because of vasoconstriction caused by catecholamines, the organs may loose perfusion.

Hypothalamus controls body temperature, and the failure of the hypothalamus may result in hypothermia.

There are a number of different strategies suggested in the literature for maintaining organs after brain death. The fact that it is possible with prolonged somatic support has been reported for a pregnant woman with brain death. By full ventilatory and nutritional support, vasoactive drugs, maintenance of normothermia, hormone replacement and other supportive measures, the fetus could be born several weeks after brain death of the mother, thereby improving the survival prognosis for the fetus.

A review article by Kenneth E. Wood and John McCartney entitled: "Management of the potential organ donor" published in Transplantation Reviews 21(2007), pages 204-218, and available online at www.sciencedirect.com, summarizes the state of the art in this field.

In Sweden, it is permitted to maintain the donor for 24 hours after brain death until organ harvesting is performed. After harvesting, the organs are examined for viability and stored, normally under hypothermic conditions until transplantation.

Since ADH decreases rapidly, it is necessary to address this issue. Depletion of ADH sooner of later results in diabetes insipidus with high urine volumes leading to hypovolemia. This has been counteracted by infusion of large volumes of colloidal or crystalloid fluid, such as Ringer's solution. Another approach is to add a vasopressor agent, such as arginine vasopressin, desmopressin, DDAVP or Minirin.

The decrease of thyroid hormone level should also be addressed in order not to aggravate metabolism. Thus, addition of T4 and/or T3 may be appropriate.

The reduction in ACTH and cortisol may be addressed by giving methylprednisolone, or a similar agent.

In order to maintain proper perfusion of the organs, especially the kidney, it is considered that a mean arterial pressure MAP of at least 60 mmHg should be upheld, see for example the above-mentioned review article. This may be done by adding catecholamines, such as nor-adrenaline and/or adrenaline. However, there is evidence that addition of adrenaline and/or nor-adrenaline may aggravate the conditions for some organs, and there is a tendency in the art to avoid the addition of catecholamines. Traditionally, dopamine has been the inotrope of choice in doses titrated to ensure cardiac output and vasoconstriction to ensure perfusion pressure gradients to the myocardium and the renal circulation.

Catecholamines have a half-life of approximately a few minutes when circulating in blood. Normal secretion in the adrenal medulla of adrenaline is 0.2 µg/kg/min and of nor-adrenaline 0.05 µg/kg/min.

It is reported in the literature that administration of nor-adrenaline has been associated with myocardial damage and initial nonfunctioning after cardiac transplantation. It is hypothesized that the "catecholamine storm" after brain death may cause myocardial ischemia or rapid desensitization of the beta-adrenergic signaling pathway. Administration of further nor-adrenaline after brain death may further desensitize the myocardial beta-adrenergic signaling.

Another possible explanation might be that, under massive catecholamine release, the uptake and inactivation metabolization systems may be saturated, resulting in a down-regulation of beta adrenergic cardiac receptors (BAR), i.e. a reduction of BAR density, which may be dose dependent. The recovery potential of BAR remains unknown, but may have an impact on organ function.

In addition, catecholamines may sulfoconjugate, which is regarded as an inactivation process by which the organism "pools" free plasma catecholamines into inactivated derivates, which subsequently are deconjutaged and released.

Thus, there is evidence that high levels of catecholamines may impair the alfa- and/or beta-receptors potency. In addition, the elimination system may be saturated, which may finally result in poor graft outcome.

A fundamental idea of the present embodiments is to replace at least some of the substances and/or hormones that are no longer excreted, or are excreted in substantially lower levels, by the brain dead body compared to a living body. The focus is to maintain hemodynamic stability by cardiovascular support because it may maintain all of the donor organs in the best possible condition.

The inventor has found that adrenaline and nor-adrenaline are two substances that would be beneficial to add, but the addition of either of the substances is controversial and may result in undesired side effects as mentioned above.

Although the exact mechanism is unknown today, it is believed that a high level of catecholamines, such as under the "catecholamine storm" will cause a depletion of the stores of catecholamine normally found in the nerve terminals and adrenal medulla. In addition, the vascular tonus is lost, because the nerve terminals receive no signals from the brain.

Nor-adrenaline is normally produced in the pre-synaptic nerve terminal from tyrosine, which is an amino acid present all over the body in large quantities.

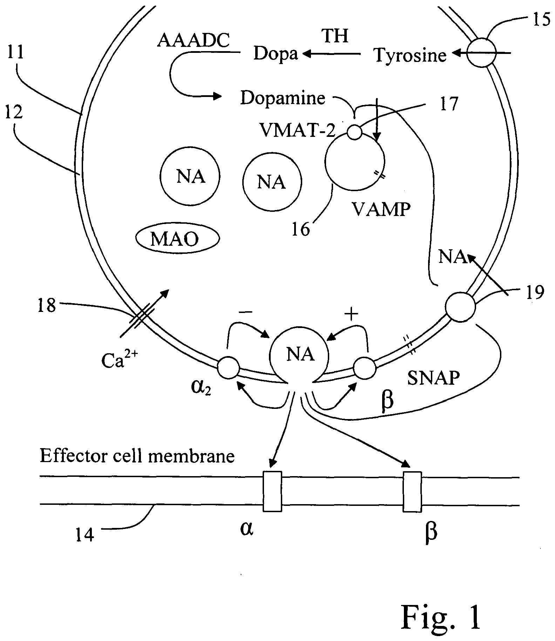

Fig. 1 is a schematic and simplified view showing a nerve terminal of the sympathetic nerve system. The nerve terminal ends in a presynaptic adrenergic varicosity 11 having a cell membrane 12. A postsynaptic effector cell membrane 14 is positioned a short distance from the cell membrane 12. The distance is called the synaptic cleft and may be about 20 nm in a chemical synapse.

Tyrosine is transported into the varicosity 11 via a transporter 15 and into the cytoplasma, wherein the tyrosine is converted to DOPA under the influence of an enzyme; Tyrosine Hydroxylase (TH). This step is considered to be the rate-limiting step in the synthesis of nor-adrenaline and adrenaline.

DOPA is transformed to dopamine in the cytoplasma under the influence of an enzyme; Aromatic L-amino acid decarboxylase (AAADC).

Dopamine is taken up into vesicles 16 via an active transporter 17 called VMAT-2 (Vesicular Monoamine Transporter), which is relatively non-specific and can transport different catecholamines, such as nor-adrenaline and dopamine, and other substances. Only about 50% of the dopamine produced is normally transported into the vesicles 16; the rest is metabolized by an enzyme called MAO (Monoamine Oxidase), see further below. There are a great number of vesicles in the nerve terminal.

Inside the vesicle, there is an enzyme; Dopamine-β-hydroxylase (DβH), which converts the dopamine entering the vesicle into nor-adrenaline (NA). In addition, any nor-adrenaline present inside the varicosity 11 is transported into the vesicle 16 by the same transporter 17 VMAT-2. In this way, nor-adrenaline is reused. A portion of the nor-adrenaline inside the varicosity does not enter the vesicle 16 but is metabolized by the enzyme MAO. Thus, there is a competition between the enzyme MAO and the active transporter 17 VMAT-2, both with regard to dopamine and nor-adrenaline.

The concentration of nor-adrenaline inside the vesicle is very high. A concentration in the range of 1 mole/liter has been reported.

At depolarization of the nerve cell membrane at the arrival of a stimulation signal, several voltage dependent calcium ion channels 18 allow the passage of calcium ions through the varicosity membrane 12. Elevated levels of calcium ions promote the fusion of vesicular membrane with the membrane of the varicosity with subsequent exocytosis of nor-adrenaline, NA. The fusion process involves the interaction of specialized proteins associated with the vesicular membrane (VAMPs, vesicle-associated membrane proteins) and the membrane of the varicosity (SNAPs, synaptiosome-associated proteins). When the vesicle emits its content into the synaptic cleft, the nor-adrenaline passes into the synaptic cleft and may interact with alfa- and beta-receptors present at the effector cell membrane, as shown by arrows in Fig. 1. Since the concentration of nor-adrenaline in the vesicle is extremely high and because the concentration of nor-adrenaline in the synaptic cleft normally is very low, and because the distance across the synaptic cleft is very small, some 20 nm, the nor-adrenaline will more or less explode due to the high concentration gradient and rapidly reaches the receptors at the effector cell membrane. The entire process comprising receipt of a depolarization voltage, inflow of calcium and exocytosis of nor-adrenaline takes often less than 1/10:th of a second.

The released nor-adrenaline may also interact with presynaptic receptors of alfa-2-type and beta-type. The alfa-2-receptor may influence directly on the vesicle and diminish the release of nor-adrenaline. The beta-receptor may facilitate the release of nor-adrenaline. The mechanism is not clearly understood for such direct influence of the release of the nor-adrenaline.

After some time, nor-adrenaline attached to the receptors is released from the receptors in the synaptic cleft. The nor-adrenaline present in the synaptic cleft is transported into the adrenal varicosity by an active transporter 19, called NET (nor-epinephrine transporter, nor-epinephrine = nor-adrenaline). This transporter has a high affinity for nor-adrenaline. NET removes free nor-adrenaline from the synaptic cleft, often within 0.1 seconds. However, a small portion of the free nor-adrenaline in the synaptic cleft passes out to the surrounding interstitial fluid and subsequently to the blood circulation. Circulating nor-adrenaline is rapidly metabolized in the liver, normally within a few minutes.

Thus, most of the nor-adrenaline released during exocytosis is reused. A portion is lost to the circulation and a portion is lost inside the adrenergic varicosity due to metabolization by MAO before entering the vesicle 16. Such lost nor-adrenaline is replaced by newly produced nor-adrenaline from tyrosine as explained above.

There is a negative feed-back regulation of the synthesis of nor-adrenaline from tyrosine. Thus, a high concentration of nor-adrenaline at the presynaptic alfa-2-receptors seems to decrease the production of nor-adrenaline, probably via interference with the rate-limiting enzyme TH.

The distance from the synaptic cleft to the blood circulation may be about 0.1 mm to several millimeters and is thus very much larger than the synaptic cleft. Thus, it takes long time for nor-adrenaline to diffuse from the synaptic cleft to the blood circulation and vice versa. Consequently, the blood concentration of nor-adrenaline in a living human body is normally low. In addition, it takes a high concentration in the blood in order for some nor-adrenaline to diffuse to the synaptic cleft and influence upon the receptors of the effector cell membrane.

Adrenaline is produced from nor-adrenaline by an extra enzymatically driven step in the adrenal medulla. The enzyme is called phenylethanolamine N-methyltransferase (PNMT) and converts nor-adrenaline to adrenaline. This enzyme is present essentially only in the adrenal medulla. The adrenal medulla comprises nerve terminals similar to the adrenergic varicosity shown in Fig. 1 but lacks a postsynaptic portion. Instead, the exocytosis takes place directly into the blood stream. Normally, the adrenal medulla excretes about 80% adrenaline and 20% nor-adrenaline into the blood.

The above description is valid for a living body, such as the human being. When the body becomes brain dead, this condition is preceded by a catecholamine storm as explained above. Thus, as much nor-adrenaline as possible is released by exocytosis in the sympathetic nerve system, causing increase of the vascular system resistance by means of the alfa-receptors of the effector cells. Consequently, all vesicles 16 in the nerve terminal are emptied into the synaptic cleft. Possibly, the uptake mechanism of the NET transporter 19 is overloaded and the concentration of nor-adrenaline in the synaptic cleft will increase, probably by a factor above 100. This will cause a disturbance of the reuse of nor-adrenaline and the reuse of the vesicles after exocytosis. In addition, there will be a down-regulation of the release of the vesicles by the alfa-2-receptors. Furthermore, the new production of nor-adrenaline will be down-regulated by the high synaptic concentration of nor-adrenaline. Most of the produced nor-adrenaline will pass out into the blood circulation and be metabolized by the liver. Consequently, all stores of nor-adrenaline in the nerve terminals will be used up and the operation of the nerve terminals will be severely disturbed.

The adrenal medulla will also release all its storage of adrenaline as well as nor-adrenaline into the blood. The circulating catecholamines will be rapidly metabolized by the liver.

Consequently, after the catecholamine storm, all stores of catecholamines, particularly nor-adrenaline and adrenaline are exhausted. There is only a small new production of nor-adrenaline because the production from tyrosine takes several hours to adjust itself to the new situation.

Thus, according to an embodiment of the present invention, adrenaline may be added in concentrations similar to those normally encountered in the blood. The added adrenaline interacts with beta-receptors to promote for example cardiac output. Adrenaline has numerous other actions in the body as is well known to the skilled person.

According to another embodiment, nor-adrenaline may be added in concentrations sufficient to cause diffusion from the blood to the synaptic cleft and to the receptors present therein, normally alfa-receptors, in order to interact with for example alfa-receptors to cause vasoconstriction. Nor-adrenaline has numerous other actions in the body as is well known to the skilled person.

However, nor-adrenaline is normally produced and normally acts at sites different from the blood. This means that nor-adrenaline in the blood circulation may have a fast and direct effect at certain locations of the body, for example the kidney, while it may take longer time and have less effect at other locations, for example the vascular system. Thus, the consequence of adding nor-adrenaline to the blood circulation is very complex and incongruous.

One mechanism which may decrease the action of the nor-adrenalin circulating in the blood and diffusing to the synaptic cleft, may be the fact that any nor-adrenaline reaching the synaptic cleft will be rapidly taken up by the NET transporter and be entered into the presynaptic nerve terminal. Since the nerve terminal is depleted of nor-adrenaline, this action will be fast. Thus, the NET transporter will compete with the activation of the effector cell receptors and decrease the action of the added circulating nor-adrenaline. In order to obtain an effect, a high concentration of nor-adrenaline in the blood will be required, which is incompatible with other operations in the body.

If nor-adrenaline is added to the blood circulation, nor-adrenaline will be absorbed or soaked up by the nerve terminals. Thus, the state of depletion of the nerve terminals after the catecholamine storm may be reversed and normal operation of the nerve terminals may be resumed. However, because the brain dead body does not emit any nerve signals, the calcium channels remain unstimulated and no exocytosis and release of nor-adrenaline may take place.

The inventor has found that the addition of cocaine together with nor-adrenaline would permit the use of much lower levels of nor-adrenaline in the blood circulation and still obtain the desired effects of vasoconstriction. One hypothesis for explaining this result may be that the cocaine acts as NET inhibitor, which is previously known. By blocking the reuptake of the nor-adrenaline from the synaptic cleft, the NET transporter will no longer compete with the alfa-receptor and the nor-adrenaline diffusing from the blood to the synaptic cleft may cause the desired action. Other explanations may be relevant in combination.

In order to test this hypothesis, other NET inhibitors were tested, such as desipramine and imipramine, which both are known to be NET inhibitors. It was shown that similar results were obtained by these tricyclic antidepressants. However, cocaine has a much smaller half-life time of about 1 hour while imipramine has a half-life time of 12 hours and desipramine has a half-life time of 30 hours. It was also shown that Venlafaxine (half-life time of 5 hours) had a similar effect. Thus, it seems that NET inhibition is at least desirable in order to potentiate the effect of blood circulation nor-adrenaline.

The conclusion is that a combination of NET inhibitors and nor-adrenaline would produce a synergetic effect in a brain dead, respirated body, in order to maintain or increase the vascular resistance and potentiate the effect of blood circulating nor-adrenaline.

At least a combination of nor-adrenaline and tricyclic antidepressants (including cocaine) would have the synergistic effect.

The same substances also have influence upon the action of adrenaline in the brain-dead, respirated body. A hypothesis is that the tricyclic antidepressants have similar effect on beta-receptors as has cocaine, for example to prevent down regulation of beta adrenergic cardiac receptors (BAR), i.e. a reduction of BAR density, see above.

Typical NET inhibitors are the following substances:
Tertiary Amine Tricyclics, such as: Amitriptyline (ELAVIL), Clomipramine (ANAFRANIL), Doxepin (ADAPIN, SINEQUAN), Imipramine (TROFANIL), Trimipramine (SURMONTIL);
Secondary Amine Tricyclics, such as: Amoxapine (ASENDIN); Desipramine (NORPRAMIN); Maprotinile (LUDIOMIL); Nortriptyline (PAMELOR); Protriptyline (VIVACTIL);
Venlafaxin (EFFEXOR); Atomexetine (WELLBUTRIN); Duloxetine (CYMBALTA); Mirtacapine (REMERON); Norclomipramine; Oxaprotiline; Lofepramine, Reboxetine, Maprotiline, Nomifensine, Doxepin, Mianserin, Viloxazine, Mirtazapine, Nisoxetine and cocaine.

The potency seems to be highest for Desipramine, Protriptylin and Norclomipramine.

In one embodiment, cocaine (benzoylmethyl ecgonine) has been used. Cocaine acts as a NET inhibitor of nor-adrenaline and dopamine. The neuronal terminals will be protected from the high systemic levels of catecholamines. The level of catecholamines in the circulating blood may be maintained by infusion of small amounts of adrenaline and/or nor-adrenaline. Thus, the neuronal action will be preserved in spite of exposure for high levels of circulating catecholamines both during the "storm" and subsequently during the next 24 hours before harvesting of the organs. By the addition of adrenaline and/or nor-adrenaline, the sympathetic and parasympathetic tones may be maintained, preventing for example uncontrolled vasodilatation and tachycardia.

Cocaine and NET inhibitors may also or alternatively act via further mechanisms not known today, and may have a beneficial effect for preserving organs before harvesting in a brain dead, respirated body.

Cocaine analogues may operate in the same way. Analogues may be any analogue as defined above. It is believed that it is the stimulant effect of cocaine that is active. Thus, cocaine analogues mean cocaine analogues with stimulating effect.

Cocaine-analogues with both stimulant & local anesthetic effects are: Dimethocaine or larocaine (DMC) ((3-diethylamino-2,2-dimethylpropyl)-4-aminobenzoate); and 3-(p-Fluorobenzoyl)tropane ((1R,5S)-(8-methyl-8-azabicyclo[3.2.1]octan-3-yl)-4-fluorobenzoate).

Cocain-analogues for stimulant effects with local anesthetic effects removed are: β-CIT (methyl (1R,2S,3S,5S)-3-(4-iodophenyl)-8-methyl-8-azabicyclo[3.2.1]octane-2-carboxylate); β-CPPIT (3β-(4'-Chlorophenyl)-2β-(3'-phenylisoxazol-5'-yl)tropane); FE-β-CPPIT (N-(2'-Fluoroethyl)-3β-(4'-chlorophenyl)-2β-(3'-phenylisoxazol-5'-yl)nortropane); FP-β-CPPIT (N-(3'-Fluoropropyl)-3β-(4-chlorophenyl)-2β-(3'-phenylisoxazol-5'-yl)nortropane); Altropane (methyl (1R,2S,3S,5S)-3-(4-fluorophenyl)-8-[(E)-3-iodoprop-2-enyl]-8-azabicyclo[3.2.1]octane-2-carboxylate); Brasofensine ((E)-1-[(1R,2R,3S,5S)-3-(3,4-dichlorophenyl)-8-methyl-8-azabicyclo[3.2.1]oct-2-yl]-N-methoxymethanimine); CFT (methyl (1R,2S,3S,5S)-3-(4-fluorophenyl)-8-methyl-8-azabicyclo[3.2.1]octane-2-carboxylate); Dichloropane (methyl (1R,2S,3S,5S)-3-(3,4-dichlorophenyl)-8-azabicyclo[3.2.1]octane-2-carboxylate); Difluoropine (methyl (1S,2S,3S,5R)-3-[bis(4-fluorophenyl)methoxy]-8-methyl-8-azabicyclo[3.2.1]octane-2-carboxylate); Ioflupane (¹²³I) (methyl (1R,2S,3S,5S)-3-(4-iodophenyl)-8-(3-fluoropropyl)-8-azabicyclo[3.2.1]octane-2-carboxylate); Nocaine (methyl (3R,4S)-4-(4-chlorophenyl)-1-methylpiperidine-3-carboxylate); Tesofensine ((1R,2R,3S,5S)-3-(3,4-dichlorophenyl)-2-(ethoxymethyl)-8-methyl-8-azabicyclo[3.2.1]octane); Troparil (methyl (1R,2S,3S,5S)-8-methyl-3-phenyl-8-azabicyclo[3.2.1]octane-2-carboxylate); Tropoxane (methyl (1R,2S,3S,5S)-3-(3,4-dichlorophenyl)-8-oxabicyclo[3.2.1]octane-2-carboxylate); (-)-Methyl-1-methyl-4β-(2-naphthyl)piperidine-3β-carboxylate (methyl (3S,4S)-1-methyl-4-naphthalen-2-ylpiperidine-3-carboxylate); PIT (2-Propanoyl-3-(4-isopropylphenyl)-tropane); PTT (2β-Propanoyl-3β-(4-tolyl)-tropane); RTI-121, IPCIT (propan-2-yl (1R,2S,3S)-3-(4-iodophenyl)-8-methyl-8-azabicyclo[3.2.1]octane-2-carboxylate); RTI-126 ((1R,2S,3S,5S)-8-methyl-2-(1,2,4-oxadiazol-5-methyl)-3-phenyl-8-azabicyclo[3.2.1]octane); RTI-150 (cyclobutyl (1R,2S,3S,5S)-8-methyl-3-(4-methylphenyl)-8-azabicyclo[3.2.1]octane-2-carboxylate); RTI-336 ((1R,2S,3S,5S)-8-methyl-2-(3-(4-methylphenyl)isoxazol-5-yl)-3-(4-chlorophenyl)-8-azabicyclo[3.2.1]octane); WF-23 (2β-Propanoyl-3β-(2-naphthyl)-tropane); WF-33 (2α-(Propanoyl)-3β-(2-(6-methoxynaphthyl))-tropane).

The use of a cocaine analogue with the stimulating effect removed and including a local anesthetic effect cannot be used for the purpose of the present embodiments. Lidocaine is a typical example. However, infusion of Lidocaine will result in vasodilatation and a lowering of the blood pressure. It is expected that other local anesthetic agents similar to Lidocaine will produce the same blood pressure lowering effect.

It has been found that by using a NET inhibitor, such as cocaine or a cocaine analogue (stimulating) or a tricyclic antidepressant, it is possible to maintain a brain-dead, heart-beating, respirated donor for 24 hours, with substantial maintenance of organ viability, by intravenous injection of the NET inhibitor and nor-adrenaline.

In particular, the heart will benefit by the NET inhibitor, which seems to decrease the cardiac irritability.

In addition, it has been found that pulmonary edema may decrease by the use of a NET inhibitor. This will improve the result of subsequent pulmonary transplantation.

By further addition of small amounts of a vasopressor agent, the operation of the kidney can be maintained and the outcome of kidney transplantation is expected to improve.

The same is true for the liver and other organs, such as pancreas, intestines, etc. This may be caused by the improved cardiovascular stability obtained.

The body is provided with proper respiratory ventilation to keep the partial pressures of oxygen and carbon dioxide at suitable levels. Normally the brain dead body has no spontaneous respiration, which means that active ventilation is required. Such ventilation may take place in any manner previously known, for example by a respirator, by external compression of thorax, by manual or mechanical means, etc.

The body is also provided with an infusion solution for maintaining fluid balance. The kidneys produce urine at a desired output level of at least 1.0 ml/kg/hour. Thus, a fluid, such as Kreb's Ringer's solution, is infused at a rate of about 1 to 5 ml/kg/hour to compensate for kidney output, sweat and fluid losses during respiration.

The composition may further contain additional components such as cortisone, thyroxin (T4), insulin, triiodotyronine (T3), a vasopressor agent, such as arginine vasopressin, desmopressin or Minirin, and methylprednisolone (cortisone).

In order to avoid diabetes insipidus, it may be proper to add desmopressin already as early as possible, for example a bolus at the start of the intervention and then a normal continuous dose as produced by the body. Desmopressin may be titrated in dependence of the urine output, in order to maintain the goal of for example 1.0 ml/kg/hour. Since the urine output immediately after the catecholamine storm is very small or even non-existent, it may be required to add a Diuretic agent, such as Furosemide (LASIX) in order to start urine production.

In one example the composition comprises the NET inhibitor, such as cocaine, and in addition adrenaline, nor-adrenaline, cortisone, thyroxin, triiodotyronine, and desmopressin.

The ratio between the NET inhibitor:nor-adrenaline may be about 1:1.

In some embodiments, the adrenaline and/or nor-adrenaline may be partly or entirely replaced by an equivalent substance. For example, phenylephrine is an alfa-1-agonist and may replace nor-adrenaline. It seems that phenylephrine is about 5 times less potent as nor-adrenaline.

Dopamine may be added in quantities less than about 0.01 mg/kg/min.

The embodiments also relate to an infusion solution comprising the composition as defined above dissolved in a pharmaceutical acceptable medium. Examples of acceptable mediums are physiological sodium chloride solution, Hartmann's solution and Ringer's (acetate) solution. Since the added volume is very small, in the range of 1.7 ml/hour (=0.04 ml/kg/hour), the ingredients may be dissolved in sterile, non-ionic water, i.e. pure H₂O.

The final amounts of the different components, which may be present in the infusion solution of a volume of 50 ml, are about 0.1 to about 10 mg of nor-adrenaline, for example 1 mg, 0.1 to about 10 mg of adrenaline, such as 1 mg, 0.1 to about 10 mg of the NET inhibitor, such as 1 mg. The other components, which may be present, may be in an amount of about 0.05 to about 3 mg of triiodotyronine, T3, about 100 to about 1000 mg hydrocortisone, insulin and desmopressin.

### EXPERIMENT 1

The following ingredients: 1 mg (1ml) adrenaline, 1 mg (1 ml) nor-adrenaline, 0.3 mg (3 ml) T3, 300 mg (3 ml) cortisone, 36 mg (9 ml) Minirin and 1 mg (3 ml) cocaine were dissolved in 50 ml physiological sodium chloride solution. The solution was added to the brain-dead, heart-beating cadaver of a pig with a weight of 40 kg, by intravenous infusion at an initial rate of 1.7 ml/hour, which was subsequently decreased in a dose dependent manner in order to maintain the mean arterial pressure MAP above about 60 mmHg. The infusion rate could be reduced to about 0.4 ml/hour over 24 hours.

In order to maintain blood volume, Macrodex replacement fluid was added at 100 ml/hour (2.5 ml/kg/hour) to support a urine output of about 1.9 liters over 24 hours. The rest of the added fluid is removed from the body via lung respiration and sweat.

In order to counteract any tendency to form edema or any vascular instability, it may be proper to add dextran to the replacement fluid, such as Dextran 40 or Dextran 70.

For reducing the risk of diabetes insipidus, a bolus of 12 mg Minirin was given at the start of the treatment.

The cadaver showed no significant signs of organ dysfunction. The heart, lungs, liver, kidney, and other organs were proper for transplantation purpose.

The blood pressure in an experiment with the above solution at a pig with a weight of 40 kg is shown in Fig. 1. The upper curve is the systolic pressure, the middle curve is the calculated mean arterial pressure MAP and the lower curve is the diastolic pressure. The ordinata shows the blood pressure in mmHg and the abscissa shows the time in hours.

After 14 hours, the infusion rate was increased, resulting in a clear and immediate increase of blood pressure. The infusion rate was then lowered to obtain base-line pressures. As is shown in Fig. 1, the MAP was maintained at 80 mmHg, which is sufficient for keeping all organs well perfused of blood.

During the harvesting of the organs after 24 hours, the infusion of the solution was again increased to baseline of 1.7 ml/hour during half an hour before harvesting and maintained until all organs were removed.

An infusion rate of 1.7 ml/hour corresponds to an infusion of 0.015 µg/kg/min of adrenaline and nor-adrenaline, which is less than the normal production rate of the adrenal medulla, which may be about 0.05 µg/kg/min. Such small infusion rate into the blood is believed to be tolerated by the organs and the system of the brain dead body.

### COMPARATIVE EXPERIMENT 2

A similar experiment was conducted wherein cocaine was replaced by desipramine. 3 mg of desipramine was added instead of 1 mg cocaine to 50 ml of physiological sodium chloride solution. In addition 1 mg of adrenaline and 1 mg of nor-adrenaline was included in the solution.

The desipramine solution was added to a pig similar to the pig in Experiment 1 at time instance 21 hours. The infusion rate was 1.7 ml/hour. The infusion rate was increased to 3.2 ml/hour at time instance 22 hours and the blood pressure started to rise. At time instance 26 hours, the infusion rate was decreased to 2.5 ml/hour. At time instance 28 hours, the infusion rate was decreased to 1.7 ml/hour.

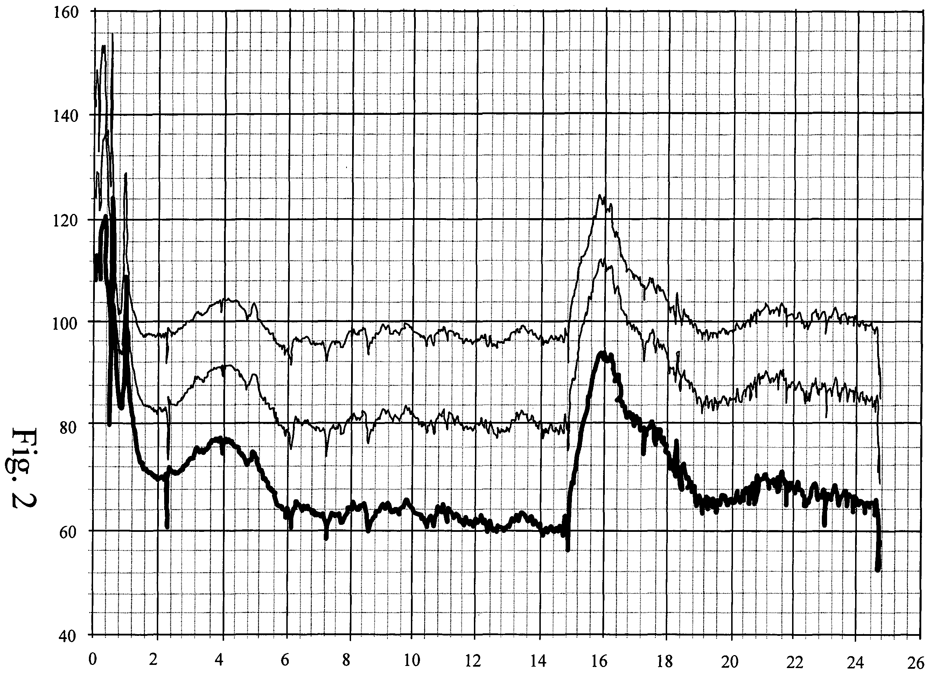

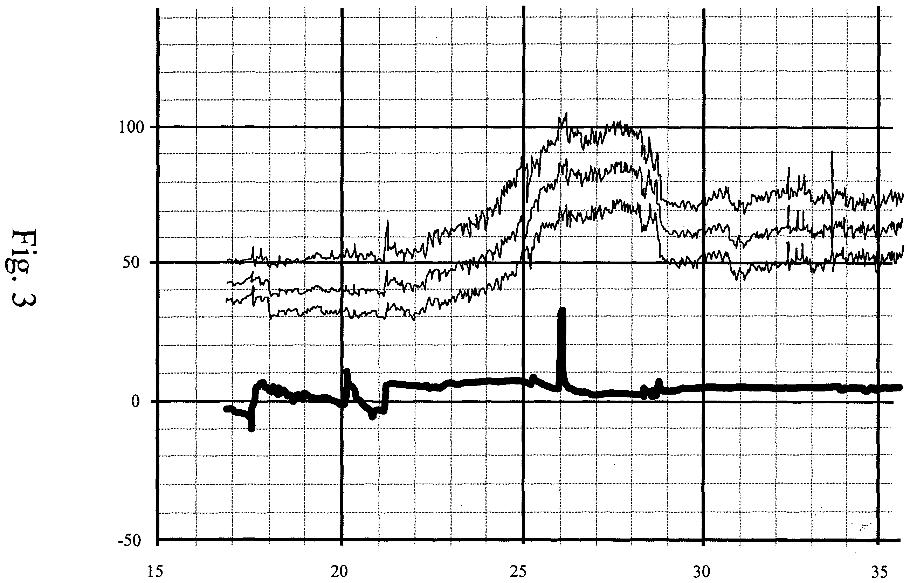

The blood pressure during the time interval from 17 hours to 35 hours is shown in Fig. 3. In the same way as Fig. 2, the upper curve is the systolic blood pressure, the middle curve is the calculated mean arterial pressure MAP and the lower curve is the diastolic pressure. In addition, the mean pulmonary pressure is shown at the bottom curve.

As can be seen, the brain-dead body answered on the infusion of desipramine by increasing the blood pressure from a mean pressure of about 40 mmHg to a mean pressure of 80 mmHg. When the infusion rate was decreased, the blood pressure went down to about 60 mmHg, but never decreased further. It is believed that desipramine maintained its NET inhibitory effect over a long time because of its long half-life time. Thus, desipramine was active even when the infusion of desipramine was decreased and for several hours thereafter.

### COMPARATIVE EXPERIMENT 3

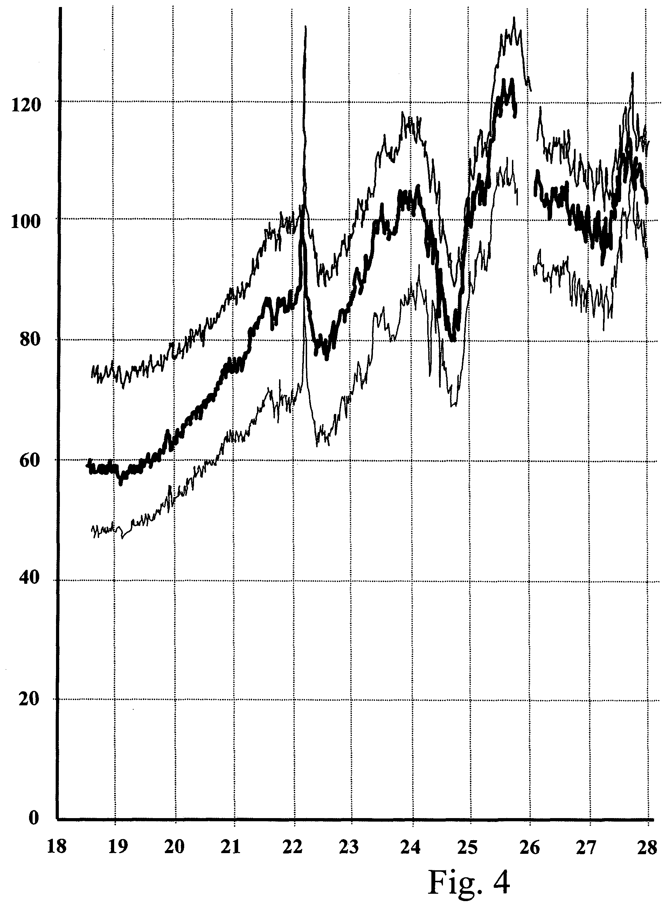

A similar experiment was conducted wherein cocaine was replaced by imipramine. In this experiment, adrenaline and nor-adrenaline was added at a constant rate of 0.4 ml/hour. At time instance 21 hours, a bolus of 2 mg cocaine was added and resulted in an increase of the blood pressure. The effect of the bolus of cocaine ceased after about 3 hours and a bolus of imipramine was added at time instance 24.5 hours. The bolus of imipramine resulted in an increase of blood pressure from about 80 mmHg to about 100 mmHg. This is shown in the diagram of Fig. 4, which however is blurred by the fact that the pressure measurement tubes were clotted and need to be flushed.

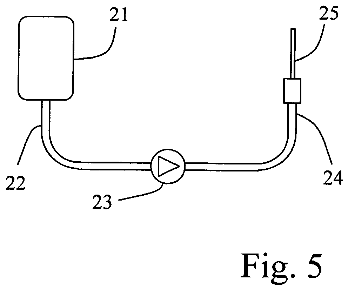

The infusion solution comprising the substances mentioned above is added to the blood circulation intravenously. The addition takes place by means of an electronically controlled infusion pump as shown in Fig. 5.

Fig. 5 shows an infusion kit according to an embodiment. The kit comprises a bag 21 for maintaining the infusion solution. The bag may comprise a volume of 50 ml of infusion solution. A tube 22 connects the bottom of the bag 21 with an infusion pump 23, which may be a syringe provided with an electric motor for automatic operation. A peristaltic pump operating on the tube 22 may alternatively be used. A second tube 24 connects the pump with a needle 25 intended to be introduced into a vein of a brain-dead, heart-beating, respirated potential donor. Alternatively, any type of access or connection to the vascular system may be used, such as a venous catheter. Since the volume to be infused over several hours is only about 50 ml, a large syringe may replace the bag and the pump.

In another embodiment, the volume of infusion solution is about 100 ml, which will be sufficient for a large patient of up to 100 kg during 24 hours. In this case, the entire procedure may take place fully automatically, without any need for manual intervention.

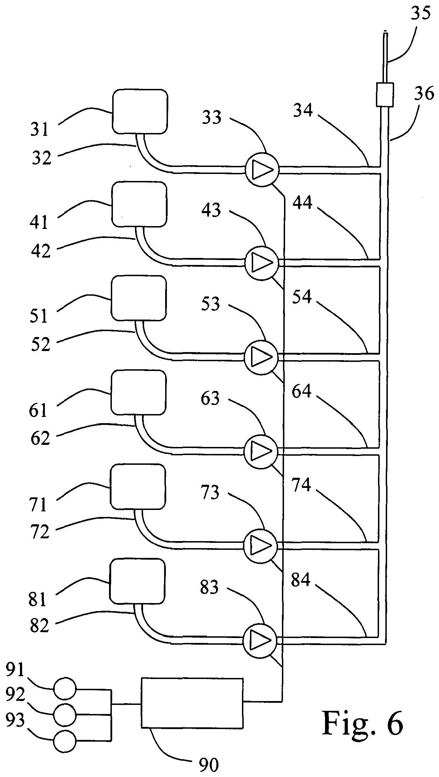

Fig. 6 discloses several infusion bags 31, 41, 51, 61, 71 and 81. The bags are the same as the corresponding bag 21 of Fig. 5. Six bags are shown in Fig. 6, but there may be any number of bags as required. A tube 32, 42, 52, 62, 72, 82 connects the bottom of the bag with an infusion pump 33, 43, 53, 63, 73, 83 which may be a syringe or a peristaltic pump. A second tube 34, 44, 54, 64, 74, 84 connects the pump with a needle 35 intended to be introduced into a vein of a brain-dead, heart-beating, respirated potential donor. A common tube 36 connects each end of the second tube to the needle 35.

Each bag may comprise one or several of the ingredients mentioned above. For example, bag 31 may comprise adrenaline, bag 41 may comprise nor-adrenaline, bag 51 may comprise cocaine or a NET inhibitor, bag 61 may comprise Minirin, bag 71 may comprise a mixture of hormones, such as T3, T4, cortisone, etc, and bag 81 may comprise an infusion solution such as Kreb-Ringer solution, which is an isotonic infusion solution comprising glucose.

Each pump may be controlled by a computer 90 as shown in Fig. 6. The computer receives input signals from sensors 91, 92, 93 etc. For example, pump 33 may be controlled in dependence of the cardiac output, for controlling addition of adrenaline, pump 43 may be controlled in dependence of the blood pressure or vascular resistance for controlling the addition of nor-adrenaline, pump 53 may be controlled in dependence of the effect of adrenaline and/or nor-adrenaline or may be controlled according to a desired predetermined control strategy, pump 63 may be controlled in dependence of the the urine output, pump 73 may be controlled according to a desired control strategy and pump 83 may be controlled for balancing the fluid loss via urine, sweat and respiration.

The computer may operate according to several strategies. One strategy may be to add cocaine at a rate which is proportional to the addition rate of nor-adrenaline. Another strategy may be to add cocaine in proportion to the addition rate of adrenaline or the sum of adrenaline and nor-adrenaline. A further strategy would be to add cocaine in a constant rate independent of the addition of adrenaline and/or nor-adrenaline.

If another NET inhibitor is used having a long half-life, a bolus at the start of the intervention would be adequate, followed by a smaller bolus after six hours etc. The effect of the bolus will be present during a long time after the bolus injection and the hemodynamic stability can be controlled by small amounts of adrenaline and nor-adrenaline.

The treatment or intervention should start as soon as possible after determination of brain death. There may be time delays between the actual death and the time when the condition of brain death is determined and such time delays should be as short as possible. The treatment may start with a bolus injection, especially if there is a substantial time delay and the blood pressure alread is below about 50 mmHg.

The treatment is continued as until it is determined that organ harvesting should be performed. Shortly before harvesting, the infusion is increased to prepare the organs for maintaining as proper condition as possible during harvesting and the time immediately following harvesting. The treatment is performed from immediately after brain death until organ harvesting.

The inventor has performed more than 20 experiments with pigs and using cocaine in combination with adrenaline and nor-adrenaline. In each case it has been possible to maintain the blood pressure at a constant level of for example 70 mmHg from the initiation of the intervention and over 24 hours. Ususally, the dose required during the first hours has to be reduced, for example from 1.7 ml/hour during the first six hours and down to 0.4 ml/hour during the last six hours. However, there are large individual differences. By this action, the blood pressure immediately after the catecholamine storm can be maintained at a sufficient level, thereby avoiding a blood pressure below 40 mmHg, which normally occurs after the catecholamine storm if no intervention is performed.

In many cases, it is not required to control all parameters and use six (or more) individually controlled supplies. One versatile combination would be to have adrenaline and cocaine in bag 31, nor-adrenaline and cocaine in bag 41, Minirin in bag 51, infusion solution in bag 61 and the other ingredients in bag 71, and excluding bag 81.

A further embodiment would include adrenaline, nor-adrenaline and cocaine in bag 31, further ingredients in a second bag 41 and infusion solution in a third bag 51.

Alternatively, both the infusion solution, which comprises a NET inhibitor, and the replacement fluid may be arranged in the same bag, which in this case should have a volume of about 2.5 liter.

In the claims, the term "comprises/comprising" does not exclude the presence of other elements or steps. Furthermore, although individually listed, a plurality of means, elements or method steps may be implemented by e.g. a single unit. Additionally, although individual features may be included in different claims or embodiments, these may possibly advantageously be combined, and the inclusion in different claims does not imply that a combination of features is not feasible and/or advantageous. In addition, singular references do not exclude a plurality. The terms "a", "an", "first", "second" etc do not preclude a plurality. Reference signs in the claims are provided merely as a clarifying example and shall not be construed as limiting the scope of the claims in any way.

Although the present invention has been described above with reference to specific embodiment and experiments, it is not intended to be limited to the specific form set forth herein. Rather, the invention is limited only by the accompanying claims and, other embodiments than those specified above are equally possible within the scope of these appended claims.

## Claims

1. Use of an infusion solution for intravascular infusion in the treatment of a brain-dead, heart-beating, respirated potential organ donor for maintaining hemodynamic stability, wherein the infusion solution comprises adrenaline, nor-adrenaline and a NET inhibitor selected from cocaine or a cocaine analogue wherein nor-adrenaline administration is between 0.08 µg/kg/h and 0.8 µg/kg/h, and wherein the cocaine analogue is selected from Dimethocaine or larocaine (DMC) ((3-diethylamino-2,2-dimethylpropyl)-4-aminobenzoate); 3-(p-Fluorobenzoyl)tropane ((1R,5S)-(8-methyl-8-azabicyclo[3.2.1]octan-3-yl)-4-fluorobenzoate); β-CIT (methyl (1R,2S,3S,5S)-3-(4-iodophenyl)-8-methyl-8-azabicyclo[3.2.1]octane-2-carboxylate); β-CPPIT (3β-(4'-Chlorophenyl)-2β-(3'-phenylisoxazol-5'-yl)tropane); FE-β-CPPIT (N-(2'-Fluoroethyl)-3β-(4'-chlorophenyl)-2β-(3'-phenylisoxazol-5'-yl)nortropane); FP-β-CPPIT (N-(3'-Fluoropropyl)-3β-(4'-chlorophenyl)-2β-(3'-phenylisoxazol-5'-yl)nortropane); Altropane (methyl (1R,2S,3S,5S)-3-(4-fluorophenyl)-8-[(E)-3-iodoprop-2-enyl]-8-azabicyclo[3.2.1]octane-2-carboxylate); Brasofensine ((E)-1-[(1R,2R,3S,5S)-3-(3,4-dichlorophenyl)-8-methyl-8-azabicyclo[3.2.1]oct-2-yl]-N-methoxymethanimine); CFT (methyl (lR,2S,3S,5S)-3-(4-fluorophenyl)-8-methyl- 8-azabicyclo[3.2.1]octane-2-carboxylate); Dichloropane (methyl (1R,2S,3S,5S)-3-(3,4-dichlorophenyl)-8-azabicyclo[3.2.1]octane-2-carboxylate); Difluoropine (methyl (1S,2S,3S,5R)-3-[bis(4-fluorophenyl)methoxy]-8-methyl-8-azabicyclo[3.2.1]octane-2-carboxylate); Ioflupane (¹²³I) (methyl (lR,2S,3S,5S)-3-(4-iodophenyl)-8-(3-fluoropropyl)-8-azabicyclo[3.2.1]octane-2-carboxylate); Nocaine (methyl (3R,4S)-4-(4-chlorophenyl)-1-methylpiperidine-3-carboxylate); Tesofensine ((1R,2R,3S,5S)-3-(3,4-dichlorophenyl)-2-(ethoxymethyl)-8-methyl-8-azabicyclo[3.2.1]octane); Troparil (methyl (1R,2S,3S,5S)-8-methyl-3-phenyl -8-azabicyclo[3.2.1]octane-2-carboxylate); Tropoxane (methyl (lR,2S,3S,5S)-3-(3,4-dichlorophenyl)-8-oxabicyclo[3.2.1]octane-2-carboxylate); (-)-Methyl-1-methyl-4β-(2-naphthyl)piperidine-3β-carboxylate (methyl (3S,4S)-1-methyl-4-naphthalen-2-ylpiperidine-3-carboxylate); PIT (2-Propanoyl-3-(4-isopropylphenyl)-tropane); PTT (2β-Propanoyl-3β-(4-tolyl)-tropane); RTI-121, IPCIT (propan-2-yl (1R,2S,3S)-3-(4-iodophenyl)-8-methyl-8-azabicyclo[3.2.1]octane-2-carboxylate); RTI-126 ((1R,2S,3S,5S)-8-methyl-2-(1,2,4-oxadiazol-5-methyl)-3-phenyl-8-azabicyclo[3.2.1]octane); RTI-150 (cyclobutyl (1R,2S,3S,5S)-8-methyl-3-(4-methylphenyl)-8-azabicyclo[3.2.1]octane-2-carboxylate); RTI-336 ((1R,2S,3S,5S)-8-methyl-2-(3-(4-methylphenyl)isoxazol-5-yl)-3-(4-chlorophenyl)-8-azabicyclo[3.2.1]octane); WF-23 (2β-Propanoyl-3β-(2-naphthyl)-tropane); or WF-33 (2α-(Propanoyl)-3β-(2-(6-methoxynaphthyl))-tropane).

2. The use of the infusion solution according to claim 1, wherein the NET inhibitor administration is between 0.08 µg/kg/h and 0.8 µg/kg/h.

3. The use of the infusion solution according to claim 1 or 2, wherein the adrenaline administration is between 0.08 µg/kg/h and 0.8 µg/kg/h.

4. The use of the infusion solution according to any one of the previous claims, wherein NET inhibitor is present in an amount of 0.2 to 5 times of the amount of nor-adrenaline.

5. The use of the infusion solution according to any one of the previous claims, wherein a ratio between cocaine and nor-adrenaline is about 1:1.

6. The use of the infusion solution according to any one of the previous claims, wherein the infusion solution further comprises at least one of: hydrocortisone, thyroxin, insulin, triiodotyronine, dopamine, desmopressin, and methylprednisolone.

7. An infusion solution for intravascular infusion for the treatment of a brain-dead, heart beating, respirated potential organ donor for maintaining hemodynamic stability, wherein the infusion solution comprises adrenaline, nor-adrenaline and cocaine, and wherein the weight ratios between adrenaline, nor-adrenaline and cocaine is 1:1:1.

8. A method for treatment of a brain dead, heart-beating, respirated potential organ donor for maintaining hemodynamic stability, comprising:
infusion of an infusion solution comprising adrenaline, nor-adrenaline and a NET inhibitor selected from cocaine or a cocaine analogue, wherein nor-adrenaline administration is between 0.08 µg/kg/h and 0.8 µg/kg/h, and wherein the cocaine analogue is selected from Dimethocaine or larocaine (DMC) ((3-diethylamino-2,2-dimethylpropyl)-4-aminobenzoate); 3-(p-Fluorobenzoyl)tropane ((1R,5S)-(8-methyl-8-azabicyclo[3.2.1]octan-3-yl)-4-fluorobenzoate); β-CIT (methyl (1R,2S,3S,5S)-3-(4-iodophenyl)-8-methyl-8-azabicyclo[3.2.1]octane-2-carboxylate); β-CPPIT (3β-(4'-Chlorophenyl)-2β-(3'-phenylisoxazol-5'-yl)tropane); FE-β-CPPIT (N-(2'-Fluoroethyl)-3β-(4'-chlorophenyl)-2β-(3'-phenylisoxazol-5'-yl)nortropane); FP-β-CPPIT (N-(3'-Fluoropropyl)-3β-(4'-chlorophenyl)-2β-(3'-phenylisoxazol-5'-yl)nortropane); Altropane (methyl (1R,2S,3S,5S)-3-(4-fluorophenyl)-8-[(E)-3-iodoprop-2-enyl]-8-azabicyclo[3.2.1]octane-2-carboxylate); Brasofensine ((E)-1-[(1R,2R,3S,5S)-3-(3,4-dichlorophenyl)-8-methyl-8-azabicyclo[3.2.1]oct-2-yl]-N-methoxymethanimine); CFT (methyl (lR,2S,3S,5S)-3-(4-fluorophenyl)-8-methyl- 8-azabicyclo[3.2.1]octane-2-carboxylate); Dichloropane (methyl (1R,2S,3S,5S)-3-(3,4-dichlorophenyl)-8-azabicyclo[3.2.1]octane-2-carboxylate); Difluoropine (methyl (1S,2S,3S,5R)-3-[bis(4-fluorophenyl)methoxy]-8-methyl-8-azabicyclo[3.2.1]octane-2-carboxylate); Ioflupane (¹²³I) (methyl (lR,2S,3S,5S)-3-(4-iodophenyl)-8-(3-fluoropropyl)-8-azabicyclo[3.2.1]octane-2-carboxylate); Nocaine (methyl (3R,4S)-4-(4-chlorophenyl)-1-methylpiperidine-3-carboxylate); Tesofensine ((1R,2R,3S,5S)-3-(3,4-dichlorophenyl)-2-(ethoxymethyl)-8-methyl-8-azabicyclo[3.2.1]octane); Troparil (methyl (1R,2S,3S,5S)-8-methyl-3-phenyl -8-azabicyclo[3.2.1]octane-2-carboxylate); Tropoxane (methyl (lR,2S,3S,5S)-3-(3,4-dichlorophenyl)-8-oxabicyclo[3.2.1]octane-2-carboxylate); (-)-Methyl-1-methyl-4β-(2-naphthyl)piperidine-3β-carboxylate (methyl (3S,4S)-1-methyl-4-naphthalen-2-ylpiperidine-3-carboxylate); PIT (2-Propanoyl-3-(4-isopropylphenyl)-tropane); PTT (2β-Propanoyl-3β-(4-tolyl)-tropane); RTI-121, IPCIT (propan-2-yl (1R,2S,3S)-3-(4-iodophenyl)-8-methyl-8-azabicyclo[3.2.1]octane-2-carboxylate); RTI-126 ((1R,2S,3S,5S)-8-methyl-2-(1,2,4-oxadiazol-5-methyl)-3-phenyl-8-azabicyclo[3.2.1]octane); RTI-150 (cyclobutyl (1R,2S,3S,5S)-8-methyl-3-(4-methylphenyl)-8-azabicyclo[3.2.1]octane-2-carboxylate); RTI-336 ((1R,2S,3S,5S)-8-methyl-2-(3-(4-methylphenyl)isoxazol-5-yl)-3-(4-chlorophenyl)-8-azabicyclo[3.2.1]octane); WF-23 (2β-Propanoyl-3β-(2-naphthyl)-tropane); or WF-33 (2α-(Propanoyl)-3β-(2-(6-methoxynaphthyl))-tropane).

9. The method according to claim 8, wherein the NET inhibitor is present in an amount of 0.2 to 5 times the amount of nor-adrenaline.

10. The method according to claims 8 or 9, wherein a ratio between cocaine and nor-adrenaline is about 1:1.

11. The method according to claim 8, 9 or 10, wherein the infusion solution further comprises at least one of: hydrocortisone, thyroxin, insulin, triiodotyronine, dopamine, desmopressin, and methylprednisolone.

12. The method according to any one of claims 8 to 11, wherein the infusion solution comprises adrenaline in the same concentration as nor-adrenaline.

13. A kit for intravenous administration for treatment of a brain dead, heart-beating, respirated potential organ donor, comprising
an infusion bag (21) comprising an infusion solution comprising adrenaline, nor-adrenaline and a NET inhibitor selected from cocaine or a cocaine analogue, and wherein nor-adrenaline has a concentration of 2 µg/ml to 200 µg/ml;
an infusion pump (23) for pumping the infusion solution to a needle (25) inserted into the vascular system of the donor for controlling the amount of infusion solution entered into the donor and tubings (22, 24) for interconnecting the bag, pump and needle,
wherein the cocaine analogue is selected from Dimethocaine or larocaine (DMC) ((3-diethylamino-2,2-dimethylpropyl)-4-aminobenzoate); 3-(p-Fluorobenzoyl)tropane ((1R,5S)-(8-methyl-8-azabicyclo[3.2.1]octan-3-yl)-4-fluorobenzoate); β-CIT (methyl (1R,2S,3S,5S)-3-(4-iodophenyl)-8-methyl -8-azabicyclo[3.2.1]octane-2-carboxylate); β-CPPIT (3β-(4'-Chlorophenyl)-2β-(3'-phenylisoxazol-5'-yl)tropane); FE-β-CPPIT (N-(2'-Fluoroethyl)-3β-(4'-chlorophenyl)-2β-(3'-phenylisoxazol-5'-yl)nortropane); FP-β-CPPIT (N-(3'-Fluoropropyl)-3β-(4'-chlorophenyl)-2β-(3'-phenylisoxazol-5'-yl)nortropane); Altropane (methyl (1R,2S,3S,5S)-3-(4-fluorophenyl)-8-[(E)-3-iodoprop-2-enyl]-8-azabicyclo[3.2.1]octane-2-carboxylate); Brasofensine ((E)-1-[(1R,2R,3S,5S)-3-(3,4-dichlorophenyl)-8-methyl-8-azabicyclo[3.2.1]oct-2-yl]-N-methoxymethanimine); CFT (methyl (1R,2S,3S,5S)-3-(4-fluorophenyl)-8-methyl- 8-azabicyclo[3.2.1]octane-2-carboxylate); Dichloropane (methyl (1R,2S,3S,5S)-3-(3,4-dichlorophenyl)-8-azabicyclo[3.2.1]octane-2-carboxylate); Difluoropine (methyl (1S,2S,3S,5R)-3-[bis(4-fluorophenyl)methoxy]-8-methyl-8-azabicyclo[3.2.1]octane-2-carboxylate); Ioflupane (¹²³I) (methyl (1R,2S,3S,5S)-3-(4-iodophenyl)-8-(3-fluoropropyl)-8-azabicyclo[3.2.1]octane-2-carboxylate); Nocaine (methyl (3R,4S)-4-(4-chlorophenyl)-1-methylpiperidine-3-carboxylate); Tesofensine ((1R,2R,3S,5S)-3-(3,4-dichlorophenyl)-2-(ethoxymethyl)-8-methyl-8-azabicyclo[3.2.1]octane); Troparil (methyl (1R,2S,3S,5S)-8-methyl-3-phenyl-8-azabicyclo[3.2.1]octane-2-carboxylate); Tropoxane (methyl (1R,2S,3S,5S)-3-(3,4-dichlorophenyl)-8-oxabicyclo[3.2.1]octane-2-carboxylate); (-)-Methyl-1-methyl-4β-(2-naphthyl)piperidine-3β-carboxylate (methyl (3S,4S)-1-methyl-4-naphthalen-2-ylpiperidine-3-carboxylate); PIT (2-Propanoyl-3-(4-isopropylphenyl)-tropane); PTT (2β-Propanoyl-3β-(4-tolyl)-tropane); RTI-121, IPCIT (propan-2-yl (1R,2S,3S)-3-(4-iodophenyl)-8-methyl-8-azabicyclo[3.2.1]octane-2-carboxylate); RTI-126 ((1R,2S,3S,5S)-8-methyl-2-(1,2,4-oxadiazol-5-methyl)-3-phenyl-8-azabicyclo[3.2.1]octane); RTI-150 (cyclobutyl (1R,2S,3S,5S)-8-methyl-3-(4-methylphenyl)-8-azabicyclo[3.2.1]octane-2-carboxylate); RTI-336 ((1R,2S,3S,5S)-8-methyl-2-(3-(4-methylphenyl)isoxazol-5-yl)-3-(4-chlorophenyl)-8-azabicyclo[3.2.1]octane); WF-23 (2β-Propanoyl-3β-(2-naphthyl)-tropane); or WF-33 (2α-(Propanoyl)-3β-(2-(6-methoxynaphthyl))-tropane)..

14. A method of infusing a solution into the circulation system of a brain dead, heart-beating, respirated potential organ donor, comprising:
determining that the donor is brain dead;
continuing or initiating respiration for oxygenation of the blood;
infusion of an infusion solution according to claim 7;
controlling the infusion by means of a computer.

## Patentansprüche

1. Verwendung einer Infusionslösung zur intravasalen Infusion bei der Behandlung eines hirntoten, herzaktiven, beatmeten potentiellen Organspenders zur Erhaltung der hämodynamischen Stabilität, wobei die Infusionslösung Adrenalin, Noradrenalin und einen aus Kokain oder einem Kokainanalogon ausgewählten NET-Inhibitor enthält, wobei die Noradrenalin-Gabe zwischen 0,08 µg/kg/h und 0,8 µg/kg/h liegt und wobei das Kokainanalogon aus
Dimethocain oder Larocain (DMC) ((3-Diethylamino-2,2-dimethylpropyl)-4-aminobenzoat),
3-(p-Fluorbenzoyl)tropan((1R,5S)-(8-Methyl-8-azabicyclo[3.2.1]oktan-3-yl)-4-fluorbenzoat), β-CIT (Methyl(1R,2S,3S,5S)-3-(4-iodphenyl)-8-methyl-8-azabicyclo[3.2.1]oktan-2-carboxylat),
β-CPPIT (3β-(4'-Chlorphenyl)-2β-(3'-phenylisoxazol-5'-yl)tropan),
FE-β-CPPIT (N-(2'-Fluorethyl)-3β-(4'-chlorphenyl)-2β-(3'-phenylisoxazol-5'-yl)nortropan), FP-β-CPPIT (N-(3'-Fluorpropyl)-3β-(4'-chlorphenyl)-2β-(3'-phenylisoxazol-5'-yl)nortropan), Altropan (Methyl(1R,2S,3S,5S)-3-(4-f|uorpheny|)-8-[(E)-3-iodprop-2-eny|]-8-azabicyclo[3.2.1]oktan-2-carboxylat),
Brasofensin ((E)-1-[(1R,2R,3S,5S)-3-(3,4-dichlorphenyl)-8-methyl-8-azabicyclo[3.2.1]okt-2-yl]-N-methoxymethanimin),
CFT (Methyl(1R,2S,3S,5S)-3-(4-fluorphenyl)-8-methyl-8-azabicyclo[3.2.1]oktan-2-carboxylat), Dichloropan (Methyl(1R,2S,3S,5S)-3-(3,4-dichlorphenyl)-8-azabicyclo[3.2.1]oktan-2-carboxylat),
Difluoropin (Methyl (1S,2S,3S,5R)-3-[bis(4-fluorphenyl)methoxy]-8-methyl-8-azabicyclo[3.2.1]oktan-2-carboxylat),
Ioflupan (¹²³I)(Methyl(1R,2S,3S,5S)-3-(4-iodphenyl)-8-(3-fluorpropyl)-8-azabicyclo[3.2.1]oktan-2-carboxylat),
Nocain (Methyl(3R,4S)-4-(4-chlorphenyl)-1-methylpiperidin-3-carboxylat),
Tesofensin ((1R,2R,3S,5S)-3-(3,4-Dichlorphenyl)-2-(ethoxymethyl)-8-methy1-8-azabicyclo[3.2.1]oktan),
Troparil (Methyl(1R,2S,3S,5S)-8-methyl-3-phenyl-8-azabicyclo[3.2.1]oktan-2-carboxylat), Tropoxan (Methy|(1R,2S,3S,5S)-3-(3,4-dichlorphenyl)-8-oxabicyclo[3.2.1]oktan-2-carboxylat), (-)-Methyl-1-methyl-4β-(2-naphthyl)piperidin-3β-carboxylat (Methyl(3S,4S)-1-methyl-4-naphthalin-2-ylpiperidin-3-carboxylat),
PIT (2-Propanoyl-3-(4-isopropylphenyl)-tropan),
PTT (2β-Propanoyl-3β-(4-tolyl)-tropan),
RTI-121,
IPCIT (Propan-2-yl(1R,2S,3S)-3-(4-iodphenyl)-8-methyl-8-azabicyclo[3.2.1]oktan-2-carboxylat),
RTI-126 ((1R,2S,3S,5S)-8-Methyl-2-(1,2,4-oxadiazol-5-methyl)-3-phenyl-8-azabicyclo[3.2.1]oktan),
RTI-150 (Cyclobutyl(1R,2S,3S,5S)-8-methyl-3-(4-methylphenyl)-8-azabicyclo[3.2.1]oktan-2-carboxylat),
RTI-336 ((1R,2S,3S,5S)-8-Methyl-2-(3-(4-methylphenyl)isoxazol-5-yl)-3-(4-chlorphenyl)-8-azabicyclo[3.2.1]oktan),
WF-23 (2β-Propanoyl-3β-(2-naphthyl)-tropan) oder
WF-33 (2a-(Propanoyl)-3β-(2-(6-methoxynaphthyl))-tropan) ausgewählt ist.

2. Verwendung der Infusionslösung nach Anspruch 1, wobei die NET-Inhibitor-Gabe zwischen 0,08 µg/kg/h und 0,8 µg/kg/h ist.

3. Verwendung der Infusionslösung nach Anspruch 1 oder 2, wobei die Adrenalingabe zwischen 0,08 µg/kg/h und 0,8 µg/kg/h ist.

4. Verwendung der Infusionslösung nach einem der vorangehenden Ansprüche, wobei der NET-Inhibitor in einer Menge von 0,2 bis 5-mal der Menge an Noradrenalin vorliegt.

5. Verwendung der Infusionslösung nach einem der vorangehenden Ansprüche, wobei das Verhältnis zwischen Kokain und Noradrenalin etwa 1:1 beträgt.

6. Verwendung der Infusionslösung nach einem der vorangehenden Ansprüche, wobei die Infusionslösung ferner mindestens einen der Stoffe Hydrocortison, Thyroxin, Insulin, Triiodtyronin, Dopamin, Desmopressin und Methylprednisolon enthält.

7. Infusionslösung zur intravasalen Infusion für die Behandlung eines hirntoten, herzaktiven, beatmeten potentiellen Organspenders zur Erhaltung der hämodynamischen Stabilität, wobei die Infusionslösung Adrenalin, Noradrenalin und Kokain enthält und wobei das Gewichtsverhältnis zwischen Adrenalin, Noradrenalin und Kokain 1:1:1 beträgt.

8. Verfahren zur Behandlung eines hirntoten, herzaktiven, beatmeten potentiellen Organspenders zur Erhaltung der hämodynamischen Stabilität, umfassend die Infusion einer Infusionslösung, welche Adrenalin, Noradrenalin und einen aus Kokain oder einem Kokainanalogon ausgewählten NET-Inhibitor enthält, wobei die Noradrenalin-Gabe zwischen 0,08 µg/kg/h und 0,8 µg/kg/h liegt und wobei das Kokainanalogon aus Dimethocain oder Larocain (DMC) ((3-Diethylamino-2,2-dimethylpropyl)-4-aminobenzoat),
3-(p-Fluorbenzoyl)tropan((1R,5S)-(8-Methyl-8-azabicyclo[3.2.1]oktan-3-yl)-4-fluorbenzoat), β-CIT (Methyl(1R,2S,3S,5S)-3-(4-iodphenyl)-8-methyl-8-azabicyclo[3.2.1]oktan-2-carboxylat),
β-CPPIT (3β-(4'-Chlorphenyl)-2β-(3'-phenylisoxazol-5'-yl)tropan),
FE-β-CPPIT (N-(2'-Fluorethyl)-3β-(4'-chlorphenyl)-2β-(3'-phenylisoxazol-5'-yl)nortropan), FP-β-CPPIT (N-(3'-Fluorpropyl)-3β-(4'-chlorphenyl)- 2β-(3'-phenylisoxazol-5'-yl)nortropan), Altropan (Methy|(1R,2S,3S,5S)-3-(4-fluorphenyl)-8-[(E)-3-iodprop-2-enyl]-8-azabicyclo[3.2.1]oktan-2-carboxylat),
Brasofensin ((E)-1-[(1R,2R,3S,5S)-3-(3,4-dichlorphenyl)-8-methyl-8-azabicyclo[3.2.1]okt-2-yl]-N-methoxymethanimin),
CFT (Methyl(1R,2S,3S,5S)-3-(4-fluorphenyl)-8-methyl-8-azabicyclo[3.2.1]oktan-2-carboxylat), Dichloropan (Methyl(1R,2S,3S,5S)-3-(3,4-dichlorphenyl)-8-azabicyclo[3.2.1]oktan-2-carboxylat),
Difluoropin (Methyl (1S,2S,3S,5R)-3-[bis(4-fluorphenyl)methoxy]-8-methyl-8-azabicyclo[3.2.1]oktan-2-carboxylat),
Ioflupan (¹²³I)(Methyl(1R,2S,3S,5S)-3-(4-iodphenyl)-8-(3-fluorpropyl)-8-azabicyclo[3.2.1]oktan-2-carboxylat),
Nocain (Methyl(3R,4S)-4-(4-chlorphenyl)-1-methylpiperidin-3-carboxylat),
Tesofensin ((1R,2R,3S,5S)-3-(3,4-Dichlorphenyl)-2-(ethoxymethyl)-8-methy1-8-azabicyclo[3.2.1]oktan),
Troparil (Methyl(1R,2S,3S,5S)-8-methyl-3-phenyl-8-azabicyclo[3.2.1]oktan-2-carboxylat),
Tropoxan (Methy|(1R,2S,3S,5S)-3-(3,4-dichlorphenyl)-8-oxabicyclo[3.2.1]oktan-2-carboxylat), (-)-Methyl-1-methyl-4β-(2-naphthyl)piperidin-3β-carboxylat (Methyl(3S,4S)-1-methyl-4-naphthalin-2-ylpiperidin-3-carboxylat),
PIT (2-Propanoyl-3-(4-isopropylphenyl)-tropan),
PTT (2β-Propanoyl-3β-(4-tolyl)-tropan),
RTI-121,
IPCIT (Propan-2-yl(1R,2S,3S)-3-(4-iodphenyl)-8-methyl-8-azabicyclo[3.2.1]oktan-2-carboxylat),
RTI-126 ((1R,2S,3S,5S)-8-Methyl-2-(1,2,4-oxadiazol-5-methyl)-3-phenyl-8-azabicyclo[3.2.1]oktan),
RTI-150 (Cyclobutyl(1R,2S,3S,5S)-8-methyl-3-(4-methylphenyl)-8-azabicyclo[3.2.1]oktan-2-carboxylat),
RTI-336 ((1R,2S,3S,5S)-8-Methyl-2-(3-(4-methylphenyl)isoxazol-5-yl)-3-(4-chlorphenyl)-8-azabicyclo[3.2.1]oktan),
WF-23 (2β-Propanoyl-3β-(2-naphthyl)-tropan) oder
WF-33 (2α-(Propanoyl)-3β-(2-(6-methoxynaphthyl))-tropan) ausgewählt ist.

9. Verfahren nach Anspruch 8, wobei der NET-Inhibitor in einer Menge von 0,2 bis 5-mal der Menge an Noradrenalin vorliegt.

10. Verfahren nach Anspruch 8 oder 9, wobei das Verhältnis zwischen Kokain und Noradrenalin etwa 1:1 beträgt.

11. Verfahren nach Anspruch 8, 9 oder 10, wobei die Infusionslösung ferner mindestens einen der Stoffe Hydrocortison, Thyroxin, Insulin, Triiodtyronin, Dopamin, Desmopressin und Methylprednisolon enthält.

12. Verfahren nach einem der Ansprüche 8 bis 11, wobei die Infusionslösung Adrenalin in derselben Konzentration wie Noradrenalin enthält.

13. Satz zur intravenösen Gabe für die Behandlung eines hirntoten, herzaktiven, beatmeten potentiellen Organspenders, umfassend
- einen Infusionsbeutel (21) mit einer Infusionslösung, welche Adrenalin, Noradrenalin und einen aus Kokain oder einem Kokainanalogon ausgewählten NTT-Inhibitor enthält und wobei Noradrenalin eine Konzentration von 2 µg/ml bis 200 µg/ml hat,
- eine Infusionspumpe (23) zum Pumpen der Infusionslösung zu einer in das Gefäßsystem des Spenders eingesetzten Kanüle (25), um die Menge der dem Spender zugeführten Infusionslösung zu steuern, und ein Schlauchsystem (22, 24) zum Verbinden von Beutel, Pumpe und Kanüle miteinander, wobei das Kokainanalogon aus
Dimethocain oder Larocain (DMC) ((3-Diethylamino-2,2-dimethylpropyl)-4-aminobenzoat),
3-(p-Fluorbenzoyl)tropan((1R,5S)-(8-Methyl-8-azabicyclo[3.2.1]oktan-3-yl)-4-fluorbenzoat), β-CIT (Methyl(1R,2S,3S,5S)-3-(4-iodphenyl)-8-methyl-8-azabicyclo[3.2.1]oktan-2-carboxylat),
β-CPPIT (3β-(4'-Chlorphenyl)-2β-(3'-phenylisoxazol-5'-yl)tropan),
FE-β-CPPIT (N-(2'-Fluorethyl)-3β-(4'-chlorphenyl)-2β-(3'-phenylisoxazol-5'-yl)nortropan), FP-β-CPPIT (N-(3'-Fluorpropyl)-3β-(4'-chlorphenyl)-2β-(3'-phenylisoxazol-5'-yl)nortropan), Altropan (Methyl(1R,2S,3S,5S)-3-(4-fluorphenyl)-8-[(E)-3-iodprop-2-enyl]-8-azabicyclo[3.2.1]oktan-2-carboxylat),
Brasofensin ((E)-1-[(1R,2R,3S,5S)-3-(3,4-dichlorphenyl)-8-methyl-8-azabicyclo[3.2.1]okt-2-yl]-N-methoxymethanimin),
CFT (Methyl(1R,2S,3S,5S)-3-(4-fluorphenyl)-8-methyl-8-azabicyclo[3.2.1]oktan-2-carboxylat),
Dichloropan (Methyl(1R,2S,3S,5S)-3-(3,4-dichlorphenyl)-8-azabicyclo[3.2.1]oktan-2-carboxylat),
Difluoropin (Methyl (1S,2S,3S,5R)-3-[bis(4-fluorphenyl)methoxy]-8-methyl-8-azabicyclo[3.2.1]oktan-2-carboxylat),
Ioflupan (¹²³I)(Methyl(1R,2S,3S,5S)-3-(4-iodphenyl)-8-(3-fluorpropyl)-8-azabicyclo[3.2.1]oktan-2-carboxylat),
Nocain (Methyl(3R,4S)-4-(4-chlorphenyl)-1-methylpiperidin-3-carboxylat),
Tesofensin ((1R,2R,3S,5S)-3-(3,4-Dichlorphenyl)-2-(ethoxymethyl)-8-methy1-8-azabicyclo[3.2.1]oktan),
Troparil (Methyl(1R,2S,3S,5S)-8-methyl-3-phenyl-8-azabicyclo[3.2.1]oktan-2-carboxylat),
Tropoxan (Methy|(1R,2S,3S,5S)-3-(3,4-dichlorphenyl)-8-oxabicyclo[3.2.1]oktan-2-carboxylat), (-)-Methyl-1-methyl-4β-(2-naphthyl)piperidin-3β-carboxylat (Methyl(3S,4S)-1-methyl-4-naphthalin-2-ylpiperidin-3-carboxylat),
PIT (2-Propanoyl-3-(4-isopropylphenyl)-tropan),
PTT (2β-Propanoyl-3β-(4-tolyl)-tropan),
RTI-121,
IPCIT (Propan-2-yl(1R,2S,3S)-3-(4-iodphenyl)-8-methyl-8-azabicyclo[3.2.1]oktan-2-carboxylat),
RTI-126 ((1R,2S,3S,5S)-8-Methyl-2-(1,2,4-oxadiazol-5-methyl)-3-phenyl-8-azabicyclo[3.2.1]oktan),
RTI-150 (Cyclobutyl(1R,2S,3S,5S)-8-methyl-3-(4-methylphenyl)-8-azabicyclo[3.2.1]oktan-2-carboxylat),
RTI-336 ((1R,2S,3S,5S)-8-Methyl-2-(3-(4-methylphenyl)isoxazol-5-yl)-3-(4-chlorphenyl)-8-azabicyclo[3.2.1]oktan),
WF-23 (2β-Propanoyl-3β-(2-naphthyl)-tropan) oder
WF-33 (2a-(Propanoyl)-3β-(2-(6-methoxynaphthyl))-tropan) ausgewählt ist.

14. Verfahren zum Infundieren einer Lösung in das Kreislaufsystem eines hirntoten, herzaktiven, beatmeten potentiellen Organspenders, umfassend
- Feststellen, dass der Spender hirntot ist,
- Fortführen oder Einleiten der Beatmung zur Sauerstoffversorgung des Bluts,
- Infusion einer Infusionslösung nach Anspruch 7,
- Steuern der Infusion mittels eines Computers.

## Revendications

1. Utilisation d'une solution de perfusion pour permettre une perfusion intravasculaire lors du traitement d'un donneur d'organe potentiel qui respire en état de mort cérébrale et dont le coeur bat pour permettre d'en maintenir la stabilité hémodynamique, la solution de perfusion renfermant de l'adréline de la nor-adrénaline et un inhibiteur de NET choisi parmi la cocaïne et un analogue de la cocaïne, l'administration de nor-adrénaline étant effectuée à raison d'entre 0.08 µg/kg/h et 0,8 µg/kg/h, et l'analogue de la cocaïne étant choisi parmi les composés suivants : dimethocaine ou larocaine (DMC) ((3-diethylamino-2,2-dimethylpropyl)-4-aminobenzoate) ;3-(p-Fluorobenzoyl)tropane ((1R,5S)-(8-methyl-8-azabicyclo[3.2.1]octan-3-yl)-4-fluorobenzoate) ; β-CIT (methyl (1R,2S,3S,5S)-3-(4-iodophenyl)-8-methyl-8-azabicyclo [3.2.1]octane-2-carboxylate) ; β-CPPIT (3β-(4'-Chlorophenyl)-2-(3'-phenyllisoxazol-5'-yl)tropane) ; FE-β-CPPIT (N-(2'-Fluoroethyl)-3β-(4'-chlorophenyl)-2β-(3'-phenylisoxazol-5'-yl)nortropane) ; FP-β-CPPIT (N-(3'-Fluoropropyl)-3β-(4'-chlorophenyl)-2β-(3'-phenylisoxazol-5'-yl)nortropane) ; Altropane (methyl (lR,2S,3S,5S)-3-(4-fluorophenyl)-8-[(E)-3-iodoprop-2-enyl]-8-azabicyclo[3.2.1]octane-2-carboxylate) ; Brasofensine ((E)-1-[(1R,2R,3S,5S)-3-(3,4-dichlorophenyl)-8-methyl-8-azabicyclo[3.2.1]oct-2-yl]-N-methoxymethanimine) ; CFT (methyl (1R,2S,3S,5S)-3-(4-fluorophenyl)-8-methyl-8-azabicyclo[3.2.1]octane-2-carboxylate) ; Dichloropane (methyl (1R,2S,3S,5S)-3-(3,4-dichlorophenyl)-8-azabicyclo[3.2.1]octane-2-carboxylate); Difluoropine (methyl (1S,2S,3S,5R)-3-[bis(4-fluorophenyl)methoxy]-8-methyl-8-azabicyclo[3.2.1]octane-2-carboxylate) ; Ioflupane (¹²³I) (methyl (1R,2S,3S,5S)-3-(4-iodophenyl-8-(3-fluoropropyl)-8-azabicyclo [3.2.1]octane-2-carboxylate) ; Nocaine (methyl (3R,4S)-4-(4-chlorophenyl)-1-methylpiperidine-3-carboxylate); Tesofensine ((1R,2R,3S,5S)-3-(3,4-dichlorophenyl)-2-(ethoxymethyl)-8-methyl-8-azabicyclo[3.2.1]octane) ; Troparil (methyl (1R,2S,3S,5S)-8-methyl-3-phenyl-8-azabicyclo[3.2.1]octane-2-carboxylate) ; Tropoxane (methyl (1R,2S,3S,5S)-3-(3,4-dichlorophenyl)-8-oxabicyclo[3.2.1]octane-2-carboxylate) ; (-)-Methyl-1-methyl-4β-(2-naphthyl)piperidine-3β-carboxylate (methyl (3S,4S)-1-methyl-4-naphthalen-2-ylpiperidine-3-carboxylate) ; PIT (2-Propanoyl-3-(4-isopropylphenyl)-tropane) ; PTT (2β-Propanoyl-3β-(4-tolyl)-tropane) ; RTI-121, IPCIT (propan-2-yl (1R,2S,3S)-3-(4-iodophenyl)-8-methyl-8-azabicyclo[3.2.1]octane-2-carboxylate) ; RTI-126 ((1R,2S,3S,5S)-8-methyl-2-(1,2,4-ocadiazol-5-methyl)-3-phenyl-8-azabicyclo[3.2.1]octane) ; RTI-150 (cyclobutyl (1R,2S,3S,5S)-8-methyl-3-(4-methylphenyl)-8-azabicyclo[3.2.]octane-2-carboxylate) ; RTI-336 ((lR,2S,3S,5S)-8-methyl-2-(3-(4-methylphenyl)isoxazol-5-yl)-3-(4-chlorophenyl)-8-azabicyclo[3.2.1]octane) ; WF-23 (2β-Propanoyl-3β-(2-naphthyl)-tropane) ; ou WF-33 (2α-(Propanoyl)-3β-(2-(6-methoxynaphthyl))-tropane).

2. Utilisation d'une solution de perfusion conforme à la revendication 1,
dans laquelle l'administration de l'inhibiteur du NET est effectuée à raison d'entre 0,08 µg/kg/h et 0,8 µg/kg/h.

3. Utilisation d'une solution de perfusion conforme à la revendication 1 ou 2,
dans laquelle l'administration d'adrénaline est effectuée à raison d'entre 0,08 µg/kg/h et 0,8 µg/kg/h.

4. Utilisation d'une solution de perfusion conforme à une quelconque des revendications précédentes,
dans laquelle l'inhibiteur du NET est présent en quantité de 0.2 à 5 fois la quantité de nor-adrénaline.

5. Utilisation d'une solution de perfusion conforme à une quelconque des revendications précédentes,
dans laquelle le rapport entre la cocaïne et la nor-adrénaline est d'environ 1:1.

6. Utilisation d'une solution de perfusion conforme à l'une quelconque des revendications précédentes,
dans laquelle la solution de perfusion renferme en outre au moins l'un des composés suivants : hydrocortisone, thyroxine, insuline, triiodotyronine, dopamine, desmopressin et methylprednisolone.

7. Solution de perfusion destinée à permettre une perfusion intravasculaire pour le traitement d'un donneur d'organe potentiel qui respire dont le coeur bat et en état de mort cérébrale pour permettre d'en maintenir la stabilité hémodynamique, la solution de perfusion renfermant de l'adrénaline, de la nor-adrénaline et de la cocaïne, et le rapport pondéral entre l'adrénaline, la nor-adrénaline et la cocaïne étant égale à 1 :1 :1.

8. Procédé de traitement d'un donneur d'organe potentiel qui respire dont le coeur bat et en état de mort cérébrale pour permettre d'en maintenir la stabilité hémodynamique, comprenant des étapes consistant à : effectuer la perfusion d'une solution de perfusion renfermant de l'adrénaline, de la nor-adrénaline et un inhibiteur du NET choisi parmi la cocaïne ou un analogue de la cocaïne, l'administration de la nor-adrénaline étant effectuée à raison d'entre 0,08 µg/kg/h et 0,8 µg/kg/h et l'analogue de la cocaïne étant choisi parmi les composés suivants : Dimethocaine ou larocaine (DMC) ((3-diethylamino-2,2-dimethylpropryl)-4-aminobenzoate) ; 3-(p-Fluorobenzoyl)tropane ((1R,5S)-(8-methyl-8-azabicyclo[3.2.1]octan-3-yl)-4-fluorobenzoate) ; β-CIT (methyl (1R,2S,3S,5S)-3-(4-iodophenyl)-8-methyl-8-azabicyclo[3.2.1]octane-2-carboxylate) ; β-CPPIT (3β-(4'-Chlorophenyl)-2β-(3'-phenylisoxazol-5'-yl)tropane); FE-β-CPPIT (N-(2'-Fluoroethyl)-3β-(4'-chlorophenyl)-2β-(3'-phenylisoxazol-5'-yl)nortropane) ; FP-β-CPPIT (N-(3'-Fluoropropyl)-3β-(4'-chlorophenyl)-2β-(3'-phenylisoxazol-5'-yl)nortropane) ; Altropane (methyl (lR,2S,3S,5S)-3-(4-flueorophenyl)-8-[(E)-3-iodoprop-2-enyl]-8-azabicyclo(3.2.1]octane-2-carboxylate) ; Brasofensine ((E)-1-[(1R,2R,3S,5S)-3-(3,4-dichlorophenyl)-8-methyl-8-azabicyclo[3.2.1]octane-2-yl]-N-methoxymethanimine) ; CFT (methyl (lR,2S,3S,5S)-3-(4-fluorophenyl)-8-methyl-8-azabicyclo(3.2.1]octane-2-carboxylate) ; Dichloropane (methyl (1R,2S,3S,5S)-3-(3,4-dichlorophenyl)-8-azabicyclo[3.2.1]octane-2-carboxylate) ; Difluoropine (methyl (1S,2S,3S,5S)-3-[bis(4-fluorophenyl)methoxy]-8-methyl-8-azabicyclo[3.2.1]octane-2-carboxylate) ; Ioflupane (¹²³I) (methyl (1R,2S,3S,5S)-3-(4-iodophenyl)-8-(3-fluoropropyl)-8-azabicyclo[3.2.1]octane-2-carboxylate) ; Nocaine (methyl (3R,4S)-4-(4-chlorophenyl)-1-methylpiperidine-3-carboxylate) ; Tesofensine ((1R,2R,3S,5S,)-3-(3,4-dichlorophenyl)-2-(ethoxymethyl)-8-methyl-8-azabicyclo[3.2.1]octane) ; Troparil (methyl (1R,2S,3S,5S)-8-methyl-3-phenyl -8-azabicyclo[3.2.1]octane-2-carboxylate) ; Tropoxane (methyl (1R,2S,3S,5S)-3-(3,4-dichlorophenyl)-8-oxabicyclo[3.2.1]octane-2-carboxylate) ; (-)-Methyl-1-methyl-4β-(2-naphthyl)piperidine-3β-carboxylate (methyl (3S,4S)-1-methyl-4-naphthalen-2-ylpiperidine-3-carboxylate) ; PIT (2-Propanoyl-3-(4-isoproprylphenyl)-tropane) ; PTT (2β-Propanoyl-3β-(4-tolyl)-tropane) ; RTI-121, IPCIT(propan-2-yl-(1R,2S,3S)-3-(4-iodophenyl)-8-methyl-8-azabicyclo[3.2.1]octane-2-carboxylate) ; RTI-126 ((1R,2S,3S,5S)-8-methyl-2-(1,2,4-ocadiazol-5-methyl)-3-phenyl-8-azabicyclo[3.2.1]octane) ; RTI-150 (cyclobutyl (1R,2S,3S,5S)-8-methyl-3-(4-methylphenyl)-8-azabicyclo[3.2.1]octane-2-carboxylate) ; RTI-336 ((lR,2S,3S,5S)-8-methyl-2-(3-(4-methylphenyl)isoxazol-5-yl)-3-(4-chlorophenyl)-8-azabicyclo[3.2.1]octane) ; WF-23 (2β-Propanoyl-3β-(2-naphthyl)-tropane) ; ou WF-33 (2α-(Propanoyl)-3β-(2-(6-methoxynaphthyl))-tropane).

9. Procédé conforme à la revendication 8,
selon lequel l'inhibiteur du NET est présent en quantité de 0.2 à 5 fois la quantité de nor-adrénaline.

10. Procédé conforme à la revendication 8 ou 9,
selon lequel le rapport entre la cocaïne et la nor-adrénaline est égal à environ 1:1.

11. Procédé conforme à la revendication 8, 9 ou 10,
selon lequel la solution de perfusion renferme en outre au moins l'un des composés suivants : hydrocortisone, thyroxine, insuline, triiodotyronine, dopamine, desmopressin et méthylprednisolone.

12. Procédé conforme à l'une quelconque des revendications 8 à 11, selon lequel la solution de perfusion renferme de l'adrénaline à la même concentration que la nor-adrénaline.

13. Kit d'administration intraveineuse destiné au traitement d'un donneur d'organe potentiel qui respire dont le coeur bat et en état de mort cérébrale comportant :
une poche de perfusion (21) contenant une solution de perfusion renfermant de l'adrénaline, de la nor-adrénaline et un inhibiteur du NET choisi parmi la cocaïne et un analogue de la cocaïne, et la nor-adrénaline ayant une concentration de 2µg/ml à 200µg/ml,
une pompe de perfusion (23) permettant de pomper la solution de perfusion vers une aiguille (25) insérée dans le système vasculaire du donneur pour commander la quantité de solution de perfusion administrée au donneur et des tubages (22, 24) pour permettre l'interconnexion de la poche de la pompe et de l'aiguille,
l'analogue de la cocaïne étant choisi parmi les composés suivants Dimethocaine ou larocaine (DMC) ((3-diethylamino-2,2-dimethylpropryl)-4-aminobenzoate) ; 3-(p-Fluorobenzoyl)tropane ((lR,5S)-(8-methyl-8-azabicyclo[3.2.1]octan-3-yl)-4-fluorobenzoate) ; β-CIT (methyl (1R,2S,3S,5S)-3-(4-iodophenyl)-8-methyl-8-azabicyclo[3.2.1]octane-2-carboxylate) ; β-CPPIT (3β-(4'-Chlorophenyl)-2β-(3'-phenylisoxazol-5'-yl)tropane); FE-β-CPPIT (N-(2'-Fluoroethyl)-3β-(4'-chlorophenyl)-2β-(3'-phenylisoxazol-5'-yl)nortropane) ; FP-β-CPPIT (N-(3'-Fluoropropyl)-3β-(4'-chlorophenyl)-2β-(3'-phenylisoxazol-5'-yl)nortropane) ; Altropane (methyl (1R,2S,3S,5S)-3-(4-flueorophenyl)-8-[(E)-3-iodoprop-2-enyl]-8-azabicyclo(3.2.1]octane-2-carboxylate) ; Brasofensine ((E)-1-[(1R,2R,3S,5S)-3-(3,4-dichlorophenyl)-8-methyl-8-azabicyclo[3.2.1]octane-2-yl]-N-methoxymethanimine) ; CFT (methyl (lR,2S,3S,5S)-3-(4-fluorophenyl)-8-methyl-8-azabicyclo(3.2.1]octane-2-carboxylate) ; Dichloropane (methyl(1R,2S,3S,5S)-3-(3,4-dichlorophenyl)-8-azabicyclo[3.2.1]octane-2-carboxylate). Difluoropine (methyl (1S,2S,3S,5S)-3-[bis(4-fluorophenyl)methoxy]-8-methyl-8-azabicyclo[3.2.1]octane-2-carboxylate) ; Ioflupane (¹²³I) (methyl (lR,2S,3S,5S)-3-(4-iodophenyl)-8-(3-fluoropropyl)-8-azabicyclo[3.2.1]octane-2-carboxylate) ; Nocaine (methyl (3R,4S)-4-(4-chlorophenyl)-1-methylpiperidine-3-carboxylate) ; Tesofensine ((1R,2R,3S,5S,)-3-(3,4-dichlorophenyl)-2-(ethoxymethyl)-8-methyl-8-azabicyclo[3.2.1]octane) ; Troparil (methyl (1R,2S,3S,5S)-8-methyl-3-phenyl -8-azabicyclo[3.2.1]octane-2-carboxylate) ; Tropoxane (methyl (1R,2S,3S,5S)-3-(3,4-dichlorophenyl)-8-oxabicyclo[3.2.1]octane-2-carboxylate) ; (-)-Methyl-1-methyl-4β-(2-naphthyl)piperidine-3β-carboxylate (methyl (3S,4S)-1-methyl-4-naphthalen-2-ylpiperidine-3-carboxylate) ; PIT (2-Propanoyl-3-(4-isoproprylphenyl)-tropane) ; PTT (2β-Propanoyl-3β-(4-tolyl)-tropane) ; RTI-121, IPCIT(propan-2-yl-(1R,2S,3S)-3-(4-iodophenyl)-8-methyl-8-azabicyclo[3.2.1]octane-2-carboxylate) ; RTI-126 ((1R,2S,3S,5S)-8-methyl-2-(1,2,4-ocadiazol-5-methyl)-3-phenyl-8-azabicyclo[3.2.1]octane) ; RTI-150 (cyclobutyl (1R,2S,3S,5S)-8-methyl-3-(4methylphenyl)-8-azabicyclo[3.2.1]octane-2-carboxylate) ; RTI-336 ((1R,2S,3S,5S)-8-methyl-2-(3-(4-methylphenyl)isoxazol-5-yl)-3-(4-chlorophenyl)-8-azabicyclo[3.2.1]octane) ; WF-23 (2β-Propanoyl-3β-(2-naphthyl)-tropane) ; ou WF-33 (2α-(Propanoyl)-3β-(2-(6-methoxynaphthyl))-tropane).

14. Procédé de perfusion d'une solution dans le système circulatoire d'un donneur d'organe potentiel qui respire dont le coeur bat et en état de mort cérébrale comprenant des étapes consistant à :
vérifier que le donneur est en état de mort cérébrale,
poursuivre ou commencer une respiration pour permettre l'oxygénation du sang,
effectuer la perfusion d'une solution de perfusion conforme à la revendication 7,
commander la perfusion au moyen d'un ordinateur.
